(19) Europäisches Patentamt
European Patent Office
Office européen des brevets

(11) EP 2 634 269 A1

(12) # EUROPEAN PATENT APPLICATION

(43) Date of publication:
**04.09.2013 Bulletin 2013/36**

(51) Int Cl.:
***C12R 1/225*** *(2006.01)* ***A61K 35/74*** *(2006.01)*

(21) Application number: **13000103.5**

(22) Date of filing: **27.10.2010**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.10.2009 BG 11050609**

(62) Document number(s) of the earlier application(s) in accordance with Art. 76 EPC:
**10795908.2 / 2 494 027**

(71) Applicant: **"Selur Vk Holding" Eood**
**1750 Sofia (BG)**

(72) Inventors:
- **Rogelj, Irena**
  **1230 Domzàle (SI)**
- **Kostadinova, Valentina Nikolaevna**
  **Sofia 1784 (BG)**
- **Matijasic, Bojana Bogovic**
  **1000 Ljubljana (SI)**

Remarks:
This application was filed on 08-01-2013 as a divisional application to the application mentioned under INID code 62.

(54) **New strain of lactic acid bacteria and its combinations producing probiotic preparations**

(57) The disclosure refers to novel strain of *Lactobacillus delbrueckii* ssp. *bulgaricus* (Selur 19) isolated from traditional yoghurt, produced in Bulgarian mountains - Stara Planina and Rodopi and the use of this novel strain in combination with probiotic strain *Lactobacillus gasseri* K7 isolated from faeces of breast fed baby and/or *Streptococcus thermophilus* Selur 12 isolated from traditional yoghurt, produced in Bulgarian mountain - Stara Planina for the preparation of a food or feed probiotic supplements. Also, this invention relates to a prophylactic and therapeutic preparations comprising, as an effective component, different combinations of above mentioned strains and biogenics originating during fermentation of milk or whey, for maintaining the host's general health and preventing and/or treating gastrointestinal diseases. Further disclosed are procedures for preparing probiotic starter culture, probiotic Bulgarian yoghurt and probiotic food supplements based on milk or whey fermentation by above mentioned strains.

EP 2 634 269 A1

## Description

### FIELD OF THE INVENTION

The present invention concerns novel strains of *Lactobacillus delbrueckii* ssp. *bulgaricus* (Selur 19) and the use of these novel strains in combination with a probiotic strain *Lactobacillus gasseri* K7 and/or *Streptococcus thermophilus* Selur 12 for the preparation of a food or feed supplements. Also, this invention relates to a prophylactic and therapeutic preparations comprising, as an effective component, different combinations of above mentioned strains, for maintaining the host's general health and preventing and/or treating gastrointestinal diseases. Further disclosed are procedures for production of biomass and preparation of probiotic starter cultures, food or feed supplements and pharmaceutical preparations based on milk or whey fermentation by above mentioned strains.

### STATE OF THE ART

Lactic acid bacteria (LAB) are generally considered a diverse group of Gram-positive, non-sporulating bacteria, incapable of respiration, nutritional fastidious, and whose major metabolic end product is lactic acid. Nowadays LAB are industrially important organisms because of their fermentative ability as well as their health and nutritional benefits. The LAB, notably lactobacilli, which occupy important niches in the gastrointestinal tracts (GIT) are considered to offer a number of probiotic benefits to general health and well being. These benefits include a positive influence on the normal microflora, competitive exclusion of pathogens, and stimulation/modulation of mucosal immunity. But it is worth to stress that health benefits imparted by probiotic bacteria are strain specific (Shah, 2007).

By FAO/WHO (2002) definition, probiotics are live microorganisms which, when ingested in adequate amounts, deliver a measured physiological benefit, which is usually strain specific. Biogenics are defined as food components derived through microbial activity which provides health benefits without involving intestinal microflora (Mitsuoka, 2000). According to this definition the biogenics are the substances such as lactic acid, butyric acid, bioactive peptides, β-galactosidase, and exopolysaccharides produced by LAB during fermentation.

Probably, the most important and sufficiently well studied biogenics in dairy products are whey proteins and bioactive peptides. Whey proteins are involved in numerous nutritious, and physiological effects, including physical performance, recovery after exercise, and prevention of muscular atrophy, satiety and weight control, management of infections and healthy aging (Smithers, 2008). Milk proteins are a rich source of bioactive peptides. Milk protein-derived bioactive peptides are inactive within the sequence of the parent protein and can be released by enzymatic proteolysis during digestion or during fermentation of milk/whey by LAB. Once they are liberated, bioactive peptides may act in the body as regulatory compounds with different activity such as: antihypertensive, antioxidative, antithrombotic, antimicrobial and immunomodulatory (Meisel, 1998, Korhonen and Pihlanto, 2006).

Yoghurt is perhaps the most popular of all fermented milks. The belief in its beneficial influence on human health has existed in many civilizations over a long period of time. In Ayurveda, one of the oldest medical sciences that back to around 2500 BC, the consumption of yoghurt has been recommended for the maintenance of overall well-being (Chopra and Doiphode, 2002). In spite of the fact that yoghurt and consequently yoghurt bacteria *Lactobacillus delbrueckii* ssp. *bulgaricus* and *Streptococcus thermophilus* were historically connected with human health, yoghurt bacteria are not frequently mentioned as probiotic bacteria. The reason for such situation is most likely the incapability of detection of these bacteria in natural microflora of human GIT and consequently opinion of most researchers in the past that these species don't survive the GIT conditions and passage (Guarner et al., 2005). Development of genetic methods has enabled scientists to trace specific strains during passage through the GIT. Recently, yoghurt bacteria have been detected in faeces of human subjects consuming yoghurt (Mater et al., 2005, Elli et al., 2006). On the contrary Garcia-Albiach et all. (2008) suggested that changes in human faecal microbiota are mainly due to the properties of the yogurt itself, which do not require bacterial viability. In this regard, some patent applications such as eg. WO 96/20607, WO 2005/056028, and WO 2008/002484 disclose specific strains of yoghurt starter culture (*Str. thermophilus* and *Lb. bulgaricus)* with probiotic properties, their combinations and preparations for treating different GIT disorders.

Up to the 1980s, most of the *Lactobacillus* strains isolated from intestines were classified as *Lb. acidophilus,* based on their morphological and phenotypical characteristics. With the development of modem taxonomy based on molecular techniques, six distinct species within the group of previously termed *Lb. acidophilus* were identified and further separated into A (*Lb. acidophilus, Lb. amylovorus, Lb. crispatus, Lb. gallinarum*) and B (*Lb. gasseri, Lb. johnsonii)* DNA homology group. The hypervariable 16S-23S intergenic spacer regions as well as 16S rRNA gene sequences were found to be sufficiently specific for differentiation between closely related *Lactobacillus* species, either by the use of species-specific PCR primers or by DNA-sequencing (Tannock et al., 1999, Kullen et al., 2000).

Strain *Lb. gasseri* K7 was isolated from 7-days old breast fed healthy baby at the Chair of Dairy Science, Biotechnical faculty, University of Ljubljana, in the year 1996 and has been deposited in the ZIM Culture Collection of Industrial Microorganisms (Slovenia, Ljubljana), registered as WDCM810 (WFCC-MIRCEN World Data Centre for Microorganisms (WDCM)). In September 2009 has been deposited according to the Budapest Agreement at the Czech Collection of Microorganisms (CCM) with the registration number CCM 7710. Based on physiological and biochemical properties including fermentative pattern determined with API 50 CHL (BioMerieux, France), it was identified as a member of *Lb. acidophilus* group. Absence of S-layer protein, PCR with species specific primers and sequencing of V2-V3 region of 16S rDNA demonstrated that strain K7 belongs to *Lb. gasseri* species (Bogovič Matijašić and Rogelj, 2000, Čanžek Majhenič et al., 2003).

It was demonstrated that *Lb. gasseri* K7 fulfils basic criteria for probiotic strains, since it is resistant to low pH and bile, produces antimicrobial substances, including bacteriocins with a wide anti-microbial spectrum, and adheres to Caco-2 cells (Bogovič Matijašić and Rogelj, 2000, Bogovič Matijašić et al., 2003). Peculiarity of *Lb. gasseri* K7 is production of at least two bacteriocins named gassericin K7 A and gassericin K7 B whose acquired nucleotide sequences were deposited at GenBank under accession numbers EF392861 and AY307382. Both bacteriocins belong to the class of two-peptide bacteriocins (Zorič Peternel, 2007).

In addition, the strain was able to survive the passage through GI tract and to colonise the intestinal mucosa of conventional and gnotobiotic piglets at least temporarily. In gnotobiotic pigs artificially infected with enterotoxigenic *Escherichia coli, Lb. gasseri* K7 reduced to certain extent the severity of infection and beside antimicrobial activity due to production of organic acids and stimulation of immune response, the competitive exclusion of *E. coli* from intestinal mucosa was proposed as a possible mechanism for such an activity. The ability of *Lb. gasseri* K7 to prevent the colonisation of enterocytes with a non-enterotoxigenic mutant *E. coli* strain O8:K88$^+$ was confirmed on Caco-2 cells model (Bogovič Matijašić et al., 2004; 2006, Rogelj and Bogovič Matijašić, 2006). Some previous patent applications have described the isolation of variety of different bacterial strains including probiotic *Lb. gasseri* strains from baby faeces (JP04320642, JP05227946, US Patent 6596530) and human milk (WO 2004/003235, WO 2008/016214).

Furthermore, USA Patent Application US2008233104 reveals compositions and methods for application of probiotic organisms in therapeutic products. In particular, the use of one or more strains of lactic acid bacteria to control pathogenes is revealed. Bacterial strains possess resistivity or strongly reduced sensitivity to anti-microbial substances, such as antibiotics and anti-viral substances. The revealed compositions are used for treatment or prevention of bacteria-mediated infections of the gastro-intestinal tract.

International Patent Application WO8905849 reveals lactic acid bacteria isolated from the gastro-intestinal tract of pigs for their ability to survive in gastro-intestinal tract conditions, i.e. for their bile or acid tolerability. These bacteria are included in a fermented milk product intended for consumption by humans or animals to treat or prevent diseases of the gastro-intestinal tract.

NZ523010 reveals an isolated strain of lactic acid bacteria possessing probiotic activity. The strains have been chosen among *Lactobacillus, Bifidobacterium* or ***Enterococcus,*** and more precisely, *Lactobacillus reuteri* NCC2581 (CNCMI-2448), *Lactobacillus reuteri* NCC2592 (CNCM I-2450), *Lactobacillus rhamnosus* NCC2583 (CNCM1-2449), *Lactobacillus reuteri* NCC2603 (CNCMI-2451), *Lactobacillus reuteri* NCC2613 (CNCMI-2452), *Lactobacillus acidophilus* NCC2628 (CNCM 1-2453).

## SUMMARY OF THE INVENTION

The invention relates to the novel strain of *Lactobacillus delbrueckii* ssp. *bulgaricus* (*Lb. bulgaricus*) isolated from traditional yoghurt, produced in Bulgarian mountains - Stara Planina and Rodopi that display several unique characteristics. This strain is *Lb. bulgaricus* Selur 19, deposited on 02. of September 2009 according to the Budapest Agreement at the Czech Collection of Microorganism (CCM) with the registration number CCM 7713,

A second aspect of the invention relates to compositions and products containing the strain mentioned above with previously described probiotic *Lb. gasseri* K7 strain, isolated from child's faeces, with the registration number CCM 7710 and *Str. thermophilus* Selur 12, deposited on 02. of September 2009 according to the Budapest Agreement at the Czech Collection of Microorganism (CCM) with the registration number CCM 7711.

Further aspect of the invention relates to procedures for production of biomass and preparation of probiotic starter cultures, food or feed supplements and pharmaceutical preparations based on milk or whey fermentation by above mentioned strains.

Finally, a last aspect of the invention relates to the use of the strains mentioned above or any culture, composition or product containing them in the manufacture of functional food products and prophylactic preparations like food and feed supplements and therapeutic preparations for maintaining the host's general health and preventing and/or treating gastrointestinal disorders, uro-genital infections and vaginal infections.

## DEFINITIONS

According to the invention, the term "probiotic" should be understood as defined by the FAO/WHO (2002), and in particular, these are live organisms which, when ingested in appropriate quantities, bring certain physiological benefit, and are usually strain-specific.

According to the invention, the term "biogene substances" means nutrition components originating from microbial activity, which are beneficial to health status, not involving intestinal microflora (Mitsuoka, 2000). According to this definition, such substances from fermented milk are for instance, lactic acid, butyric acid, bioactive peptides, β-galactosidase and exopolysaccharides produced by LAB during fermentation.

According to the invention and, as used here, the term "nutrition and/or pharmaceutically acceptable substances/bearer" means one or more solid or liquid substances or fillers, diluents or capsulating substances, which is(are) suitable for intake by humans or animals and which is(are) compatible with the active probiotic strain.

According to the invention, the term "compatible" refers to components, which are not inhibiting to probiotic bacteria and allow chestnut colouration and strain activity according to this invention and the *Lb. gasseri* strain K7 in the human or animal body.

## BRIEF DESCRIPTION OF THE DRAWING

**Figure 1** shows PCR with *Lb. delbrueckii* species-specific Del I and II primers. Key: M - 100bp (Fermentas); 1. ; 2. L7; 3. L8; 4. L12; 5. Selur 19; 6. LMG 6412$^T$ *Lb. delbr.bulg.;* 7. LMG 6901$^T$ *Lb. delbr.delbr*.; 8-12 *Lb.delbr.bulg.* isolated from commercial yoghurts; 13. Negative control;

**Figure 2** shows PCR with *Str. thermophilus* species-specific Th1 and Th2 primers. Key: M - 100bp (Fermentas); 1 - ST4; 2 - ST7; 3 - Selur 12

**Figure 3** shows PCR with *Lb. delbrueckii* subsp. *bulgaricus* subspecies-specific LB1 and LLB1 primers. Key: M - 1kb (Fermentas); 1-; 2 - L7; 3 - L8; 4 - L12; 5 - Selur 19; 6 - LMG 6412$^T$ *Lb. delbr.bulg.;* 7 - LMG 6901$^T$ *Lb. delbr.delbr*.; 8-12 - *Lb.delbr.bulg.* isolated from commercial yoghurts; 13. Negative control.

**Figure 4** shows DNA fragments obtained after RAPD-PCR with primer 1254. Key: M - 1kb (Fermentas); I - ; 2 - L7; 3 - L8; 4 - L12; 5 - Selur 19; 6 - LMG 6412$^T$ *Lb. delbr.bulg.;* 7 - LMG 6901$^T$ *Lb. delbr.delbr*.; 8-12 - *Lb.delbr.bulg.* isolated from commercial yoghurts; 13. Negative control;

**Figure 5** shows DNA fragments obtained after RAPD-PCR with primer KGT-70GC. Key: 1+12 - Marker I kb (Fermentas); *2 - Lb. gasseri* K7; 3 - *Str. thermophilus* Selur 12; *4 - Lb. bulgaricus* Selur 6; 5 - *Lb. bulgaricus* Selur 19; 6-10 - *Lb.delbr.bulg.* isolated from commercial yoghurts; 11 - Negative control (RAPD- reaction master mix).

**Figure 6** presents antimicrobial activity of *Lb. bularicus* Selur 19 strains against *Staphylococcus aureus* ATCC 29213. *Lb. gasseri* K7 was included for comparison.;

**Figure 7** presents the adhesion ability of different strains to CaCo-2 cells; (1 = *Lb. bulgaricus* Selur 19, 2 = *Str. thermophilus* Selur 12, 3 = *Lb. bulgaricus* Selur 6, 4 = *Lb. rhamnosus* GG, 5 = *Lb. johnsonii* Lj1, 6 = *Lb. reuteri* ING 1, 7 = *Lb.* Shirota SHI, 8 = *Lb. gasseri* K7).

**Figure 8** presents the changes of pH values during fermentation of milk inoculated with *Str. thermophilus* Selur 12 and different lactobacilli strains (, Selur 19 and K7) at 42 °C and 37 °C

## DETAILED DISCLOSURE OF THE INVENTION

### Novel *Lactobacillus delbrueckii* ssp. bulgaricus strain Selur 19 and the *Streptococcus thermophilus* strain Selur 12

Samples of the novel *Lactobacillus delbrueckii* ssp. *bulgaricus* strain Selur 19 and *Streptococcus thermophilus* strain Selur 12 have been deposited at CCM (Czech Collection of Microorganism) under the accession numbers CCM 7712, CCM 7713, and CCM 7711 with a deposit date 02. of September 2009. The deposit has been made under the conditions of the Budapest Treaty on the International Recognition of the Deposit of Microorganisms for the Purposes of Patent Procedure.

*Streptococcus* strain Selur 12 was isolated from traditional yoghurt produced from raw milk at Stara Planina Mountains (Bulgaria) and *Lactobacillus* strain Selur 19 was isolated from traditional yoghurt produced from raw milk at Rodopi Mountains (Bulgaria).

Likewise a sample of the *Lactobacillus gasseri* strain K7 has been deposited at CCM (Czech Collection of Microorganism) under the accession number CCM 7710 with a deposit date of 02. of September 2009. Also this deposit has been made under the conditions of the Budapest Treaty on the International Recognition of the Deposit of Microorganisms for the Purposes of Patent Procedure. *Lb. gasseri* K7 is a well-described, probiotic strain (Bogovič Matijašić and Rogelj, 2000; Bogovič Matijašić et al., 2003, 2004, 2006; Rogelj and Bogovič Matijašić, 2006, Zorič Peternel, 2007) with the accession number IM105 in the ZIM Culture Collection of Industrial Microorganisms (Slovenia), registered as WDCM810 (WFCC-MIRCEN World Data Centre for Microorganisms (WDCM). It was isolated in August 1996 at Ljubljana, Slovenia from 7 days old breast fed infant's faeces. K7 is producer of two bacteriocins: gassericin K7 A and gassericin K7 B. On the basis of nucleotide sequences of bacteriocins' operons found on the chromosome, both bacteriocins were classified into a group of two-peptide non-lantibiotics (IIb). Nucleotide sequences of gassericina K7 A (1143 bp) and gassericin K7 B (3276 bp) are deposited in the GenBank, accession numbers EF392861 (gassericin K7A) and AY307382 (gassericin K7B). It contains I plasmid, forms shorter rods occurring in long chains. Optimal temperature for growth is 37°C in microaerophilic conditions.

As described in **Example 1** below, the novel *Lactobacillus* strain and *Streptococcus* strain were first characterized by genotyping. PCR with *Lb. delbrueckii* species specific primers Del I (5'-ACG GAT GGA TGG AGA GCA G-3') and Del II (5'-GCA AGT TTG TTC TTT CGA ACT C-3') and *Str. thermophilus* species specific primers Th I (5'-ACG GAA TGT ACT TGA GTT TC-3') and Th II (5'-TTT GGC CTT TCG ACC TAA C-3') (Tilsala-Timisjärvi and Alatossava, 1997) was performed on DNA extracted from pure cultures by Wizard® Genomic DNA Purification Kit (Promega) or by Maxwell™ 16 system (Promega). Subspecies identification of *Lb. delbrueckii* was conducted with primers LB1 (5'-AAA AAT GAA GTT GTT TAA AGT AGG TA-3') and LLB1 (5'-AAG TCT GTC CTC TGG CTG G-3') (Torriani et al., 1999). Strains and Selur 19 were identified as *Lactobacillus delbrueckii* ssp. *bulgaricus* and strain Selur 12 as *Streptococcus thermophilus.*

To additionally confirm species identification, sequencing of the PCR amplified variable regions V1 and V3 of 16S rRNA was carried out. *Lactobacillus* strain Selur 19 was identical (99%) with the reference sequence of *Lactobacillus delbrueckii* ssp. *bulgaricus* ATCC BAA-365 or ATCC 11842 ((Sequence of PCR extended variable regions V1 and V3 of 16S rRNA of *Lb. bulgaricus* strain (The sequencing was made by Microsynth Lab in Balgach - Switzerland) and Sequence of PCR extended variable regions V1 and V3 of 16S rRNA of *Lb. bulgaricus* strain Selur 19 (The sequencing was made by Microsynth Lab in Balgach - Switzerland)),

A1, Premium RUN
986445 49879
[redacted]
GATTTGTTGGACGCTAGCGGCGGATGGGTGAGTAACACGTGGGCAATCTGCCCTA
AAGACTGGGATACCACTTGGAAACAGGTGCTAATACCGGATAACAACATGAATC
GCATGATTCAAGTTTGAAAGGCGGCGTAAGCTGTCACTTTAGGATGAGCCCGCGG
CGCATTAGCTAGTTGGTGGGGTAAAGGCCTACCAAGGCAATGATGCGTAGCCGA
GTTGAGAGACTGATCGGCCACATTGGGACTGAGACACGGCCCAAACTCCTACGG
GAGGCAGCAGTAGGGAATCTTCCACAATGGACGCAAGTCTGATGGAGCAACGCC
GCGTGAGTGAAGAAGGTTTTCGGATCGTAAAGCTCTGTTGTTGGTGAAGAAGGAT
AGAGGCAGTAACTGGTCTTTATTTGACGGTAATCAACCAGAAAGTCACGGCTAAC
TACGTGCCAGCAGCCGCGGTAATACGTAGGTGGCAAGCGTTGTCCGGATTTATTG
GGCGTAAAGCGAGCGCAGGCGGAATGATAAGTCTGATGTGAAAGCCCACGGCTC
AACCGTGGAACTGCATCGGAAACTGTCATTCTTGAGTGCAGAAGAGGAGAGTGG
AATTCCATGTGTAGCGGTGA

---

Lactobacillus .................................................. 116 hits 20 orgs [root; cellular organisms; Bacteria; Firmicutes; Bacilli; Lactobacillales; Lactobacillaceae]
. Lactobacillus delbrueckii .................................... 94 hits 7 orgs
. . Lactobacillus delbrueckii subsp. bulgaricus ................ 61 hits 3 orgs
. . . Lactobacillus delbrueckii subsp. bulgaricus ATCC BAA-365 . 9 hits 1 orgs
. . . Lactobacillus delbrueckii subsp. bulgaricus ATCC 11842 ... 10 hits 1 orgs
. . Lactobacillus delbrueckii subsp. indicus .................. 1 hits 1 orgs
. . Lactobacillus delbrueckii subsp. delbrueckii .............. 4 hits 1 orgs
. . Lactobacillus delbrueckii subsp. lactis .................... 13 hits 1 orgs
. Lactobacillus sp. JCM 8653 .................................. 1 hits 1 orgs
. Lactobacillus sp. CY1 ........................................ 1 hits 1 orgs
. uncultured Lactobacillus sp. ................................ 9 hits 1 orgs [environmental samples]
. Lactobacillus sp. RA2120 .................................... 1 hits 1 orgs
. Lactobacillus sp. RA2062 .................................... 1 hits 1 orgs
. Lactobacillus sp. DJF_CR11 .................................. 1 hits 1 orgs
. Lactobacillus sp. RA2066 .................................... 1 hits 1 orgs
. Lactobacillus sp. 19-2 ...................................... 1 hits 1 orgs
. Lactobacillus sp. JCM 1552 .................................. 1 hits 1 orgs
. Lactobacillus sp. DumLac1 .................................. 1 hits 1 orgs
. Lactobacillus sp. MF-07 ..................................... 1 hits 1 orgs
. Lactobacillus sp. DI70 ...................................... 1 hits 1 orgs
. Lactobacillus helveticus .................................... 2 hits 1 orgs

---

Selur 12 strain was identical (100%) with the reference sequence of *Streptococcus thermophilus* LMG 18311 ( (Sequence of PCR extended variable regions V1 and V3 of 16S rRNA of *Str. thermophilus* strain Selur 12 (The sequencing was made by Microsynth Lab in Balgach - Switzerland)),

A1, Premium RUN
986447 49881
[redacted]
GAGTTGCGAACGGGTGAGTAACGCGTAGGTAACCTGCCTTGTAGCGGGGATAA
CTATTGGAAACGATAGCTAATACCGCATAACAATGAATGACTCATGTCATTTATT
TGAAAGGGGCAATTGCTCCACTACAAGATGGACCTGCGTTGTATTAGCTAGTAGG
TGAGGTAACGGCTCACCTAGGCGACGATACATAGCCGACCTGAGAGGGTGATCG
GCCACACTGGGACTGAGACACGGCCCAGACTCCTACGGGAGGCAGCAGTAGGGA
ATCTTCGGCAATGGGGGCAACCCTGACCGAGCAACGCCGCGTGAGTGAAGAAGG
TTTTCGGATCGTAAAGCTCTGTTGTAAGTCAAGAACGAGTGTGAGAGTGGAAAGT
TCACACTGTGACGGTAGCTTACCAGAAAGGGACGGCTAACTACGTGCCAGCAGC
CGCGGTAATACGTAGGTCCCGAGCGTTGTCCGGATTTATTGGGCGTAAAGCGAGC
GCAGGCGGTTTGATAAGTCTGAAGTTAAAGGCTGTGGCTCAACCATAGTTCGCTT
TGGAAACTGTCAAACTTGAGTGCAGAAGGGGAGAGTGGAATTCCATGTGTAGCG
GTGAAATGCG

Streptococcus .................. 100 hits 4 orgs [root; cellular organisms; Bacteria; Firmicutes; Bacilli; Lactobacillales; Streptococcaceae]
. uncultured Streptococcus sp. . 46 hits 1 orgs [environmental samples]
. Streptococcus salivarius ..... 10 hits 1 orgs
. Streptococcus sp. C165 ....... 1 hits 1 orgs
. Streptococcus thermophilus ... 43 hits 1 orgs

Strain K7 was identical (100%) with the reference sequence of *Lactobacillus gasseri* ATCC 33323 ((Sequence of PCR extended variable regions V1 and V3 of 16S rRNA of *Lb. gasseri* strain K7 (The sequencing was made by Microsynth Lab in Balgach - Switzerland)).

A1, Premium RUN
986443 49877
[redacted]
AATTTGGTGCTTGCACCAAATGAAACTAGATACAAGCGAGCGGCGGACGGTGA
GTAACACGTGGGTAACCTGCCCAAGAGACTGGGATAACACCTGGAAACAGATGC
TAATACCGGATAACAACACTAGACGCATGTCTAGAGTTTAAAAGATGGTTCTGCT
ATCACTCTTGGATGGACCTGCGGTGCATTAGCTAGTTGGTAAGGTAACGGCTTAC
CAAGGCAATGATGCATAGCCGAGTTGAGAGACTGATCGGCCACATTGGGACTGA
GACACGGCCCAAACTCCTACGGGAGGCAGCAGTAGGGAATCTTCCACAATGGAC
GCAAGTCTGATGGAGCAACGCCGCGTGAGTGAAGAAGGGTTTCGGCTCGTAAAG
CTCTGTTGGTAGTGAAGAAAGATAGAGGTAGTAACTGGCCTTTATTTGACGGTAA
TTACTTAGAAAGTCACGGCTAACTACGTGCCAGCAGCCGCGGTAATACGTAGGTG
GCAAGCGTTGTCCGGATTTATTGGGCGTAAAGCGAGTGCAGGCGGTTCAATAAGT
CTGATGTGAAAGCCTTCGGCTCAACCGGAGAATTGCATCAGAAACTGTTGAACTT
GAGTGCAGAAGAGGAGAGTGGAACTCCATGTGTAGCGGTGAAATG

Lactobacillus ........................ 111 hits 19 orgs [root; cellular organisms; Bacteria; Firmicutes; Bacilli; Lactobacillales; Lactobacillaceae]
. Lactobacillus gasseri ............. 51 hits 2 orgs
. . Lactobacillus gasseri ATCC 33323 . 7 hits 1 orgs
. Lactobacillus sp. IDSAc ............ 1 hits 1 orgs
. uncultured Lactobacillus sp. ........ 11 hits 1 orgs [environmental samples]
. Lactobacillus sp. BLB3 ............ 1 hits 1 orgs
. Lactobacillus sp. KC36a ............ 1 hits 1 orgs
. Lactobacillus sp. BLB1b ............ 1 hits 1 orgs
. Lactobacillus johnsonii ............ 34 hits 2 orgs
. . Lactobacillus johnsonii NCC 533 .. 6 hits 1 orgs
. Lactobacillus sp. BCRC 17755 ....... 1 hits 1 orgs
. Lactobacillus sp. 20326H4L1 ........ 1 hits 1 orgs
. Lactobacillus sp. DJF_SLA41 ........ 1 hits 1 orgs

| | | |
|---|---|---|
| Lactobacillus sp. AD102 ........... | 1 hits | 1 orgs |
| Lactobacillus sp. L-YJ ............ | 1 hits | 1 orgs |
| Lactobacillus sp. KC45a ........... | 2 hits | 1 orgs |
| Lactobacillus acidophilus .......... | 1 hits | 1 orgs |
| Lactobacillus sp. JCM 2010 ......... | 1 hits | 1 orgs |
| Lactobacillus sp. ID9203 .......... | 1 hits | 1 orgs |
| Lactobacillus sp. MF213 ........... | 1 hits | 1 orgs |

As further discussed in **Example 1** RAPD patterns of novel strains were determined. It is important to distinguish special strain from others and in this respect RAPD-PCR can be a useful method. For differentiation the novel strains from different strains used for industrial yoghurt production several oligonucleotide primers were used. The most appropriate for strain differentiation turned out to be RAPD primers 1254 (Fig. 4) or KGT-70GC (Fig. 5 ).

In a further aspect, the present invention describes characteristics which render the novel strain alone or in combination with *Lb. gasseri* strain K7 beneficial to human health, and in particular in the prophylaxis or treatment against digestive, infective and other immune related diseases such as allergies or inflammatory diseases, traveller's diarrhea or antibiotic associated diarrhea.

As demonstrated in **Example 2** different tests were performed according to "Final Technical report for FSA project ref G01022" (FSA, 2005) to evaluate the ability of novel strains to survive in simulated conditions of gastrointestinal tract. According to results, at pH 1 only Selur 12 survive 20 minutes treatment in SCJ in the rate comparable to the strain *Lb. gasseri* K7. At pH 2 app. half of the viable cells of all tested strains survive, but 100 % of *Lb. gasseri* K7 cells (Table 1). Strains were much more sensitive to simulated upper intestine juice consisted of Bile salts no 3 (1 g/l) in PBS buffer (pH 8) and pancreatic enzyme (1 g/L). A decrease in cell counts for three or four log units immediately after exposure to UIJ (at T 0) was observed for all tested strains in pure cultures. *Str. thermophilus* Selur 12 was not affected immediately but incubation for 2 hours was detrimental for this strain (Table 2). The survival of strains was better in preparations based on milk matrix (Table 3). According to *in vitro* tests it is unlikely that *Str. thermophilus* Selur 12 survive the GIT conditions. The important feature of this strain is its stimulating effect on the growth and fermentative ability of *Lb. bulgaricus* strain Selur19 (demonstrated in Example 9).

**In Example 3** Antibiotic susceptibility profiles of novel strains are presented. *Lactobacillus* strain Selur 19 is susceptible/sensitive to four (i.e. gentamycin, tetracycline, erythromycin and vancomycin) of the clinically most relevant antibiotics. When the Danielsen and Wind (2003) breakpoints are applied also *Lb. gasseri* is susceptible to these four antibiotics. In accordance with the FEEDAP Panel Report (European Commission, 2005) *Str. thermophilus* Selur 12 is resistant to gentamycin and erythromycin, but no breakpoint values have been suggested by CLSI/NCCLS. All strains possess streptomycin resistance according to the FEEDAP (European Commission, 2005) breakpoints. When the Danielsen and Wind (2003) breakpoints are applied, the *Lactobacillus* strain is found to be sensitive to streptomycin. Since no breakpoint values exist for metronidazole, cefotaxime, cefaclor and amoxicillin, strains cannot be indicated as sensitive or resistant. High MIC values for all strains were obtained for metronidazole. This observation is in accordance with Delgado et al. (2007) who observed intrinsic resistance to this antibiotic in most *Lactobacillus* species when tested isolates from the human gastrointestinal tract. *Str. thermophilus* Selur 12 is resistant to azithromycin when microbiological breakpoint in accordance with CLSI/NCCLS, for clinical studies is taken into consideration. No breakpoint value has been suggested for this antibiotic by FEEDAP Panel Report (European Commission, 2005).

The demonstration of some probiotic characteristics of novel strain is particularly preferred embodiment of the invention. In this respect, the novel strain that is the subject of this invention was evaluated for antibacterial activity, the hydrophobicity, β-galactosidase activity, adhesion ability to Caco-2 cells, competition and exclusion properties, and immune response of THP-1 macrophage on stimulation with *Lb. bulgaricus* strain.

Antibacterial activity was tested by deferred agar spot test against 31 indicator strains including different lactobacilli, *Clostridium perfringens* 1b 1106, *Cl. tyrobutyricum* DSM 2637, *Bacilus cereus* CCM 2010$^T$, three strains of *Staphylococcus aureus, Enterococcus durans* CCM 5612, two strains of *Enterococcus faecalis, E. faecium* LMG 11423, *Listeria monocytogenes* IM 372, *L. innocua* ATCC 33090 and *E. coli* O8:K88. As presented in **Example 4** broad range of antimicrobial activity was demonstrated especially for *Lb. bulgaricus* strain Selur 19 (Table 8). Extensive antimicrobial activity of *Lb. bulgaricus* Selur 19 strain was observed against not only *Lactobacillus* indicator strains but also against S. *aureus, L. monocytogenes* and *L. innocua. Str. thermophilus* Selur 12 in addition to lactobacilli only moderately inhibited enterococci and *Listeria* strains. The novel strain did not inhibite *Clostridium perfringens* 1b 1106, *Cl. tyrobutyricum* DSM 2637, *Enterococcus durans* CCM 5612, and *E. coli* O8:K88. Antibacterial activity of *Lb. gasseri* K7 against different strains of *Clostridium tyrobutyricum* and *Cl. difficile* was demonstrated previously (Bogovič Matijašić and Rogelj, 2000, Bogovič Matijašić et al., 2007).

Hydrophobicity was determined according to Vinderola and Reinheimer (2003) by measurement of partition of bacterial cells between organic (n-hexadecane) and aqueous phases. As indicated in **Example 5** high cell-surface hydrophobicity was determined in addition to *Lb. gasseri* K7 for *Lb. bulgaricus* Selur 19.

The highest β-galactosidase activity, evaluated by the protocol of Vinderola and Reinheimer (2003) using synthetic compound o-nitrophenyl-ß-D-galactoside (OPNG), was expressed by *Lb. bulgaricus* strain. Compared with other strains *Lb. bulgaricus* strain Selur 19 expressed an excellent β-galactosidase activity **(Example 6)** which is a very good feature for all those with impaired lactase activity.

An important functional attribute for probiotic bacteria is the ability of the strain to adhere to intestinal epithelial cells. In our experiments Caco-2 cells were used as *in vitro* model. Precise procedure is described in **Example 7** together with the test for competition and exclusion properties of *Lb. bulgaricus* strain Selur 19 against two strains, S. *aureus* ATCC 29213 and *E. coli* ATCC 25922. The adhesion of *Lb. bulgaricus* Selur 19 and *Str. thermophilus* Selur 12 strains was in general at least comparable or in the case when % of adhesion was calculated (adhered cfu/well divided with added cfu/well expressed in %) even better from that determined for *Lb. gasseri* K7 and some other probiotic strains (*Lb. rhamnosus* GG, *Lb. johnsonii* Lj1, *Lb. reuteri* ING 1, *Lb.* Shirota SHI). In competition assay *Lb. bulgaricus* Selur 19 was able to inhibit the adhesion of E. *coli* ATCC 25922 but not S. *aureus.* In the exclusion assay Selur 19 was ineffective.

Modulation of immune response is one of the beneficial health effects of some probiotic bacteria. Since some preliminary experiments indicated possible immune stimulating effect of *Lb. gasseri* K7 and *Lb. bulgaricus* strain both strains were tested for their ability to affect the secretion of cytokines by THP-1 macrophage cell line. Due to their well-known probiotic effects including modulation of immune response, *Lb. rhamnosus* GG and *Lb. casei* DN-114001 were used as a positive control. Experiment is described in **Example 8.** and K7 strains did not induce secretion of inflammatory cytokines (IL-8, IL-6 and TNF) by macrophage cell line THP-1, while both strains reduced the concentration of the majority of inflammatory cytokines induced by the addition of LPS (lipopolysaccharides of *E. coli* O111:B4). The reduced secretion of inflammatory cytokines indicates that the and K7 strains could have an anti-inflammatory effect. The observed increase in IL-1β could lead to lymphocyte B proliferation and subsequent IgA production, which has to be confirmed *in vivo.*

**Probiotic preparations consisting of the novel strain alone or in combination with *Lb. gasseri* strain K7**

Lactic acid bacteria are commonly used as starter cultures for dairy or other food industry. A starter cultures that comprise at least one of the strains of the invention is an embodiment of the invention. Typically, such a starter culture composition comprises the bacteria in a concentrated form including frozen, dried or freeze-dried concentrates typically having a concentration of viable cells, which is in the range of $10^6$ to $10^{11}$ cfu (colony forming units) per gram of the composition including at least $10^6$ cfu per gram of the composition, such as at least $10^7$ cfu/g, e. g. at least $10^8$ cfu/g, such as at least $10^9$ cfu/g, e.g. at least $10^{10}$ cfu/g, such as at least $10^{11}$ cfu/g.

It is also an aspect of invention comprising the bacterial strain of the invention together with *Lb. gasseri* K7 or another bacterial strain. In this regard, this invention refers to biological pure cultures of each of the strains, or mixtures of them or with *Lb. gasseri* K7 or with other bacterial strains. Thus, said aspect of this invention is the production of different compositions including probiotic comprising at least one strain or a mixture of the strains of the invention.

When lactic acid bacteria are used as a starter culture for production of fermented food for example fermented milk the knowledge of fermentative ability of strains and combinations that assure the high number of viable strains is essential. Study of the different combinations of strains and fermentation temperatures is described in **Example 9.** *Str. thermophilus* strain Selur 12 has been shown as a good stimulator of lactobacilli. Milk inoculated with different combinations of strains was fermented (means pH 4,6 was reached) in 5 to 7,5 hours. Very good growth and survival of probiotic *Lb. gasseri* strain K7 during fermentation of milk with *Str. thermophilus* strain Selur 12 and *Lb. bulgaricus* strain Selur 19 was observed at two temperatures of fermentation 42 °C and 37 °C, respectively. The viable count of *Lb. gasseri* K7 in fermented milk reached the level from 8,70* $10^8$ to 1,6* $10^9$ cfu/ml when fermentation was carrying at 42 °C and 37 °C, respectively. It was confirmed that *Lb. gasseri* K7 can be combined with novel strains for probiotic food products and preparations.

Useful food product starting materials include any material which is conventionally subjected to a lactic acid bacterial fermentation step such as milk, vegetable materials, meat products, fruit juices, and doughs.

The next important implementation of this invention is a technology for production of biomass and preparation of probiotic starter cultures, food or feed supplements and pharmaceutical preparations. Procedure is in detail delineated in **Example 10.** The quoted compositions can be based on fermentation of milk, whey or vegetable substrates such as soya, rice or other grain milk.

Typically, preparation based on milk fermentation which can be used also as a starter culture comprises the freeze-dried bacteria in concentration of viable cells, which is in the range of $10^7$ to $10^{10}$ cfu (colony forming units) per gram of the preparation, including at least $10^7$ cfu/g, e.g. at least $10^8$ cfu/g, e.g. at least $10^9$ cfu/g, such as at least $10^{10}$ cfu/g, less than 400 mg of lactose per gram and lactulose which is in the range of 25 to 50 mg/g.

A further aspect of the invention consists in a food product comprising support material and at least one strain according to the invention, a culture, a composition or a product according to the invention. Preferable the support material is a food selected from milk, yoghurt, cheese, fermented milks, milk based fermented products, meat based fermented products, fermented cereals based products, milk and cereals based powders, infant formulae, food and feed supplements, ice creams, juices, candies, bread, cakes and chewing-gums. In a preferred embodiment, the microbial strain of present invention is contained at the level of about $10^6$ to about $10^{11}$ cfu/g of supported material, preferably from about $10^6$ to about $10^9$ cfu/g, more preferably from about $10^7$ to about $10^9$ cfu/g of support material.

In another aspect the present invention provides probiotic food and feed supplement and pharmaceutical preparations comprising the strain according to the invention combined with Lb. *gasseri* K7 alone or with Lb. *gasseri* K7 and other nutrients, prebiotics, minerals, vitamins or other nutritional or/and pharmaceutical acceptable substances. For preparing the quoted preparations according the present invention at least one of the strains of the present invention has to be present in an amount of from $10^7$ cfu/g to about $10^{11}$ cfu/g support material and *Lb. gasseri* strain K7 has to be present in an amount of from $10^8$ cfu/g to about $10^{11}$ cfu/g support material, preferably from $10^9$ cfu/g to about $10^{11}$ cfu/g, more preferably from $10^{10}$ cfu/g to about $10^{11}$ cfu/g of supported material.

The probiotic food and feed supplement and pharmaceutical preparations can be prepared in forms of tablets, capsules, granules, liquid bacterial suspensions, paste and powder. In addition the probiotic composition of the present invention can be any ingestible material selected from the group consisting of milk, curd, milk based fermented products, acidified milk, yoghurt, frozen yoghurt, milk powder, milk based powders, milk concentrate, cheese, cheese spreads, beverages, ice-creams, fermented cereal based products, infant formulae, tablets, liquid bacterial suspensions, dried oral supplement, wet oral supplement, dry tube feeding or wet tube-feeding that is produced by the use of at least one strain of this invention in combination with *Lb. gasseri* strain K7.

In a further embodiment, the mentioned probiotic composition further comprises a nutritional or/and pharmaceutical acceptable substances and pharmaceutically acceptable carrier. As used herein, the term " nutritional or/and pharmaceutical acceptable substances/carrier" means one or more solid or liquid substance or filler diluents or encapsulating substances which are suitable for administration to a human or an animal and which is/are compatible with the active probiotic strain. The term "compatible" relates to components which are not inhibiting to probiotic bacteria and enable good survival and activity of strain of the present invention and *Lb. gasseri* strain K7 in human or animal body.

In useful embodiments, probiotic composition according to the invention is suitable for preventing or treating a disease, syndrome or condition from the group consisting of antibiotic-associated disorders, gastroenteritis, diarrhoea including traveller's diarrhoea and acute infantile diarrhoea, lactose intolerance, gastrointestinal infections and colonization of the gastrointestinal tract by pathogenic bacteria including *Clostridium difficile,* irritable bowel syndrome (IBS), inflammatory bowel disease (IBD) and other immunomodulative syndromes.

## EXAMPLES

### Example 1: Characterization of the novel *Lb. bulgaricus* and *Str. thermophilus* strains

Isolated strains were purified 3-times according to classical method on M17 (*Str. thermophilus* Selur 12) and MRS (*Lb. bulgaricus* Selur 19) agar plates and characterized by genotyping.

In addition to novel strain of *Lactobacillus delbrueckii* ssp. *bulgaricus* (Selur 19) and *Streptococcus thermophilus* Selur 12 the reference strains obtained from culture collections (LMG $6901^T$ *Lb. delbrueckii* ssp. *bulgaricus,* LMG $6412^T$ *Lb. delbrueckii* ssp. *delbrueckii),* strains isolated from traditional yoghurts (lactobacilli: L7, L8, and L12; streptococci: ST4 and ST7) and strains isolated from a range of different yoghurts obtained on market were used for comparison.

*Lactobacillus* strains were grown in MRS broth/MRS agar and *Streptococcus* strains in M17 broth/M17 agar (Merck, Darmstadt, Germany) at 42 °C. Genomic DNA was extracted from pure cultures of novel isolates and reference strains by Wizard® Genomic DNA Purification Kit (Promega) or by Maxwell™ 16 system (Promega).

Species and subspecies specific PCR

PCR with *Lb. delbrueckii* species specific primers Del I (5'-ACG GAT GGA TGG AGA GCA G-3') and Del II (5'-GCA AGT TTG TTC TTT CGA ACT C-3') and *Str. thermophilus* species specific primers Th I (5'-ACG GAA TGT ACT TGA GTT TC-3') and Th II (5'-TTT GGC CTT TCG ACC TAA C-3') (Tilsala-Timisjärvi and Alatossava, 1997) was performed on DNA extracted from pure cultures of selected strains for species identification. DNA fragments of expected cca. 200 bp were obtained after PCR with *Lb. delbrueckii* species specific Del I and Del II primers (Fig 1.) and DNA fragments of expected cca. 260 bp were obtained after PCR with *Str. thermophilus* species specific Th I and Th II primers (Fig. 2). Fragment of larger size is probably dimer and is missing in other subspecies reference strain from collection *Lb. delbrueckii* ssp. *delbrueckii* (Fig. 1).

Subspecies identification of *Lb. delbrueckii* was conducted with primers LB1 (5'-AAA AAT GAA GTT GTT TAA AGT AGG TA-3') and LLB1 (5'-AAG TCT GTC CTC TGG CTG G-3') (Torriani et al., 1999). DNA fragments of expected cca. 1065 bp were obtained after PCR with *Lb. delbrueckii* ssp. *bulgaricus* subspecies specific LB1 and LLB1 primers. No amplification products were observed in other subspecies collection strain *Lb. delbrueckii* ssp *delbrueckii* (Fig 3).

Identification of strains by sequencing of the PCR amplified variable regions V1 and V3 of 16S rRNA

For additional confirmation of species identification, sequencing of the PCR amplified variable regions V1 and V3 of 16S rRNA was done. Isolated genomic DNA of *Lb. delbrueckii* ssp. *bulgaricus* strain (Selur 19, and L7, L8, L12), *Str. thermophilus* Selur 12 strain and *Lb. gasseri* K7 strain was PCR amplified by primers P1(5'-GCG GCG TGC CTA ATA CAT GC-3') and P4 (5'-ATC TAC GCA TTT CAC CGC TAC-3') to obtain 660 bp size variable regions V1 and V3 of 16S rRNA of lactic acid bacteria to identify and classify species as previously described by Klijn et al. (1991). PCR protocol was modified in annealing (at 52 °C for 30 s) and elongation (at 72°C for 30 s) steps. PCR products were purified by agarose gel electrophoreses and extracted from the gel using Wizard® SV Gel and PCR Clean-up system (Promega). Sequencing of PCR products was done by Microsynth lab in Balgach - Switzerland. The resulting sequences were further analysed online in NCBI database by BLAST search to determine homology with nucleotides sequences from the GenBank database and consequently determine the species/subspecies.

*Lactobacillus* strain Selur 19 was identical (99%) with the reference sequence of *Lactobacillus delbrueckii* subsp. *bulgaricus* ATCC BAA-365 or ATCC 11842, Selur 12 strain was identical (100%) with the reference sequence of *Streptococcus thermophilus* LMG 18311. It was confirmed again that strain K7 was identical (100%) with the reference sequence of *Lactobacillus gasseri* ATCC 33323.

In accordance to previous results obtained by PCRs with species and subspecies specific PCR primers (Fig. 1, 2 and 3) novel strain Selur 19 was confirmed as *Lactobacillus delbrueckii* ssp. *bulgaricus* and strain Selur 12 as *Streptococcus thermophilus.*

RAPD-PCR amplification

Several oligonucleotide primers were used singly in one series of PCR amplification in order to find the best possibility to differentiate RAPD patterns between novel *Lb. delbrueckii* ssp. *bulgaricus* strains and to differentiate novel strains from different *Lb. bulgaricus* strains used for industrial yoghurt production:

- **M13** (Torriani et al.,1999) (5'-GAG GGT GGC GGT TCT-3') (2 variants of protocol were performed: protocol described by Torriani et al.(1999) - 4 mM $MgCl_2$ and annealing temperature 45 °C for 20 s - and protocol conducted by Rossetti and Giraffa (2005) - 3 mM $MgCl_2$ and annealing temperature 42 °C for 20 s),
- **OPL-01** (Van Reenen and Dicks, 1996) (5'-GGC ATG ACC T-3'),
- **VALIO** (Tynkkynen et al., 1999) (5'-AGT CAG CCA C-3'),
- **1254** (Torriani et al., 1999) (5'-CCG CAG CCA A -3'),
- **KGT-70GC*** (5'-AGC GGG CGT A-3') and
- **KGT-80GC*** (5'-CGC GTG CCC A-3') with novel protocol of 4mM $MgCl_2$ and cycling program 95°C for 2 min, 35 cycles: 95°C for 1 min, 37°C for 2 min, 72°C for 2 min and final extension 72°C for 5 min).
  *The oligonucleotide primers KGT-70GC and KGT-80GC were constructed by Chair of Dairy Science research group (University of Ljubljana, Biotechnical faculty)

Amplification products were analysed by electrophoresis in 1,5 % gels with TAE buffer and stained by SYBR Safe DNA gel stain (Molecular Probes, Invitrogen) and photographed.

The most appropriate in number, distribution, intensity of the exhibited bands for strain differentiation turned out to be RAPD primers 1254 (Fig.4) or KGT-70GC (Fig. 5).

**Example 2: Survival of novel strains in simulated conditions of gastrointestinal tract (GIT)**

Different tests were performed according to FSA (2005). In addition to Selur 19 and Selur 12 strains, *Lb. gasseri* strain K7 isolated from child faeces and *Lb. bulgaricus* strains L7, L8 and L12, isolated from traditional yoghurts were included in trials for comparison.

Survival in simulated gastric juice (SGJ)

Simulated gastric juice (SGJ) medium was made of peptone water (7.5 g/l) adjusted at pH 1, 2, 3 and 6.5 using 1 M Hydrochloric acid. Prior to the experiment, a fresh solution of pepsine (300 mg/L) was added in quantity to reach final concentration of 15 mg/L. 10 ml of overnight cultures of strains were concentrated by centrifugation and pellet re-suspended in 1 ml SGJ. At time T 0 and after 20 min, 1 ml of sample was diluted in pepton water and plated on MRS or M17 agar. Plates were incubated for 48 h at 37 °C. Results are expressed as ratio (r) between log value of viable cells after 20-min exposure to simulated SGJ and log value of viable cells before treatment. According to results, at pH I only strain Selur 12 survive 20 minutes treatment in SCJ in the rate comparable to the strain *Lb. gasseri* K7. At pH 2 app. half of the viable cells of all tested strains survive, but 100 % of *Lb. gasseri* K7 cells. pH 3 and 6,5 are partly detrimental only for *Str. thermophilus* Selur 12 (Table 1).

**Table 1.** Survival of *Lb. bulgaricus* strains L7, L8, L12, Selur 19, , *Lb. gasseri* K7, and *Str. thermophilus* Selur 12 strains in simulated gastric-intestinal juice at pH 1, 2, 3 and 6,5 for 20 min (r is average value of two tests)

| Strain | $r_{pH1}$ | $SD_{PH1}$ | $r_{pH2}$ | $SD_{pH2}$ | $r_{pH3}$ | $SD_{pH3}$ | $r_{pH6,5}$ | $SD_{pH6,5}$ |
|---|---|---|---|---|---|---|---|---|
| **L7** | 0,13 | 0,00 | 0,49 | 0,02 | 0,96 | 0,01 | 1,00 | 0,00 |
| **L8** | 0,20 | 0,01 | 0,55 | 0,01 | 1,01 | 0,01 | 1,00 | 0,01 |
| **L12** | 0,12 | 0,00 | 0,58 | 0,05 | 1,00 | 0,00 | 0,99 | 0,02 |
| **Selur 19** | 0,46 | 0,09 | 0,57 | 0,06 | 1,00 | 0,00 | 1,00 | 0,00 |
| **K7** | 0,54 | 0,00 | 1,00 | 0,01 | 0,99 | 0,01 | 0,99 | 0,01 |
| **Selur 12** | 0,00 | 0,00 | 0,55 | 0,01 | 0,86 | 0,03 | 0,96 | 0,06 |
| Key:<br>r - average of cells at T20/average of cells at T0<br>SD - standard deviation<br>r=1 - the cells survive 20-minute exposure to SGJ,<br>r>1 -growth of the strain,<br>r=0,5 - a loss of the half of viable cells present in the culture | | | | | | | | |

Survival in simulated upper intestine juice (UIJ)

Simulated upper intestine juice (UIJ) consisted of Bile salts no 3 (1 g/l) suspended in 1x PBS buffer (pH 8). Prior to the experiment, a freshly prepared solution of pancreatic enzyme (1 g/L) was added. The cells from 1 ml of overnight culture or from 1 g of the preparation with strains, was subsequently inoculated in 10 ml of UIJ and incubated at 37 °C for 2 hours. At T 0 and 2 h, 1 ml of suspension was diluted and plated on MRS or M17 agar. Plates were incubated for 48 h at 37 °C. A decrease in cell counts for three or four log units immediately after exposure to UIJ (at T 0) was observed for all tested strains in pure cultures. After 2 h treatment, additional decrease for two logs was observed only for control strain L12. *Str. thermophilus* Selur 12 was not affected immediately while incubation for 2 hours was detrimental for this strain (Table 2). When the biomass of strains were prepared by the fermentation of milk in bioreactor (described in Example 10) and lyophilized preparation with strains was exposed to UIJ, the survival of strains was much better (Table 3).

**Table 2.** Survival of *Lactobacillus* strains L7, L8, L12, Selur 19, K7 and *Str. thermophilus* Selur 12 strain in simulated UIJ after incubation at 37 °C for 2 hours (the $log_{10}$ values are averages of two tests)

| Strain | Cont. (cfu/ml) | SD | T0 | SD | T2 | SD |
|---|---|---|---|---|---|---|
| **L7** | 7,40 | 0,27 | 3,77 | 0,53 | 3,29 | 0,26 |
| **L8** | 8,23 | 0,21 | 4,01 | 0,73 | 2,78 | 1,17 |
| **L12** | 8,70 | 0,08 | 5,41 | 0,46 | 3,76 | 0,20 |
| **Selur 19** | 8,43 | 0,54 | 5,54 | 0,90 | 5,17 | 0,84 |
| **K7** | 8,68 | 0,11 | 4,80 | 0,45 | 4,20 | 0,80 |

(continued)

| Strain | Cont. (cfu/ml) | SD | T0 | SD | T2 | SD |
|---|---|---|---|---|---|---|
| **Selur 12** | 7,80 | 0,25 | 7,64 | 0,35 | 0,35 | 0,60 |
| Key:<br>Contents cfu/ml - initial number of cells (colony forming units/ml) in $log_{10}$ values<br>T0 - $log_{10}$ cells at the beginning - time 0<br>T2 - $log_{10}$ cells after 2-hours incubation<br>SD - standard deviation | | | | | | |

**Table 3.** Survival of *Lb. bulgaricus* in preparation based on milk in simulated UIJ after incubation at 37 °C for 2 hours

| Preparation | (cfu/g) | T0 (cfu/g) | T2h (cfu/g) |
|---|---|---|---|
| 1 | 8,27 | 7,51 | 6,05 |
| 2 | 9,17 | 8,33 | 6,69 |
| 3 | 9,10 | 8,24 | 6,89 |
| Key:<br>cfu/g - initial number ofcells (colony forming units/g) in $log_{10}$ values<br>T0 - $log_{10}$ cells at the beginning - time 0<br>T2 - $log_{10}$ cells after 2-hours incubation | | | |

**Example 3: Antibiotic susceptibility profiles of novel strain**

E-test® strips (AB Biodisk, Solna, Sweden) for the determination of either streptomycin (SM), gentamicin (GM), tetracycline (TC), ampicillin (AM), metronidazole (MZ), clindamycin (CM), chloramfenicol (CL), erythromycin (EM) rifampicin (RI), vancomycin (VA), cefotaxime (CTL), cefaclor (CF), amoxicillin (AC), azithromycin (AZ), clarithromycin (CH), or amoxycillin/clavulanat (XL) resistance were used according to the manufacturer's instructions. Mueller Hinton Agar (MH agar) plates with bacterial suspensions were prepared as follows: saline suspensions with cell density of approximately $10^7$ cfu/ml were prepared from overnight MRS broth cultures, and 100 $\mu$L of suspensions were spread out on dried MH agar supplemented with 10 % of MRS (Klare et al., 2005). The plates were incubated in anaerobic (lactobacilli) or aerobic (*Str. themophilus*) conditions for 24 h at 37 °C before Minimum Inhibition Concentration (MIC) was read. MIC values reported by FEEDAP Panel Report (European Commission, 2005) and Danielsen and Wind (2003) were used to classify strain as sensitive (s) or resistant (r) (Tables 4-7).

**Table 4.** Results of MIC determination by Etest® (AB Biodisk, Sweden) for *Lb. gasseri* K7 (results of two tests are presented; 1.parall., 2. parall.).

| *Lactobacillus gasseri K7* | $MIC_{bp}^{a}$ | $MIC_{bp}^{b}$ | MIC ($\mu$g/ml) | | s/r |
|---|---|---|---|---|---|
| | | | 1.paral. | 2.paral. | |
| STREPTOMYCIN (SM) (0,064-1024) | 16 | > 256 | 96 | 96 | r/s |
| GENTAMYCIN (GM) (0,064-1024) | 8 | 256 | 64 | 48 | r/s |
| TETRACYCLINE (TC) (0,016-256) | 8 | 4 | 3 | 4 | s |
| AMPICILLIN (AM) (0,016-256) | 4 | 4 | 2 | 2 | s |
| METRONIDAZOLE (MZ) (0,016-256) | - | - | > 256 | > 256 | ? |

(continued)

| *Lactobacillus gasseri K7* | $MIC_{bp}^{a}$ | $MIC_{bp}^{b}$ | MIC (μg/ml) | | s/r |
|---|---|---|---|---|---|
| | | | 1.paral. | 2.paral. | |
| CLINDAMYCIN (CM) (0,016-256) | 4 | 64 | 8 | 8 | r/s |
| CHLORAMPHENICOL (CL) (0,016-256) | 4 | 16 | 6 | 4 | r/s |
| ERYTHROMYCIN (EM) (0,016-256) | 4 | 1 | 0,19 | 0,19 | s |
| RIFAMPICIN (RI) (0,016-256) | - | 2 | 0,094 | 0,094 | s |
| VANCOMYCIN (VA) (0,016-256) | 4 | 4 | 1,5 | 2 | s |
| CEFOTAXIME (CTL) (0,02-32) | - | - | 1,5 | 2 | ? |
| CEFACLOR (CF) (0,016-256) | - | - | 16 | 16 | ? |
| AMOXICILLIN (AC) (0,016-256) | - | - | 1,5 | 1,0 | ? |
| AZITHROMYCIN (AZ) (0,016-256) | - | 8 | 1,5 | 1,5 | s |
| CLARITHROMYCIN (CH) (0,016-256) | - | 8 | 0,125 | 0,125 | s |
| AMOXYCILLIN/ CLAVULANAT (XL) (0,016-256) | - | 16/8 | 0,75 | 0,75 | s |

$MIC_{bp}^{a}$: microbiological breakpoints for obligate homofermentative species of *Lactobacillus,* in accordance with the FEEDAP Panel Report (European Commission, 2005).
$MIC_{bp}^{b}$: microbiological breakpoints in accordance with Danielsen and Wind (2003).
s/r: sensitive/resistant
- : no data
?: not indicated since no breakpoint value has been suggested for this antibiotic

Table 5. Results of MIC determination by Etest® (AB Biodisk, Sweden) for *Lb. bulgaricus* Selur 19 (results of two tests are presented; 1.parall., 2. parall.).

| *Lactobacillus bulgaricus* Selur 19 | $MIC_{bp}^{a}$ | $MIC_{bp}^{b}$ | MIC(μg/ml) | | s/r |
|---|---|---|---|---|---|
| | | | 1.parall. | 2.parall. | |
| STREPTOMYCIN (SM) (0,064-1024) | 16 | > 256 | 24 | 32 | r/s |
| GENTAMICIN (GM) (0,064-1024) | 8 | 256 | 4 | 4 | S |
| TETRACYCLINE (TC) (0,016-256) | 8 | 4 | 2 | 2 | S |
| AMPYCILLIN (AM) (0,016-256) | 4 | 4 | 0,047 | 0,064 | S |
| METRONIDAZOLE (MZ) (0,016-256) | - | - | > 256 | > 256 | ? |

(continued)

| *Lactobacillus bulgaricus* Selur 19 | $MIC_{bp}{}^{a}$ | $MIC_{bp}{}^{b}$ | MIC(μg/ml) | | s/r |
|---|---|---|---|---|---|
| | | | 1.parall. | 2.parall. | |
| CLINDAMYCIN (CM) (0,016-256) | 4 | 64 | 0,032 | 0,032 | S |
| CHLORAMPHENICOL (CL) (0,016-256) | 4 | 16 | 3 | 3 | S |
| ERYTHROMYCIN (EM) (0,016-256) | 4 | 1 | 0,064 | 0,047 | S |
| RIFAMPICYN (RI) (0,016-256) | - | 2 | 0,25 | 0,25 | S |
| VANCOMYCIN (VA) (0,016-256) | 4 | 4 | 2 | 1,5 | S |
| CEFOTAXIME (CTL) (0,02-32) | - | - | 0,5 | 0,38 | ? |
| CEFACLOR (CF) (0,016-256) | - | - | 8 | 8 | ? |
| AMOXICILLIN (AC) (0,016-256) | - | - | 0,25 | 0,38 | ? |
| AZITHROMYCIN (AZ) (0,016-256) | - | 8 | 0,5 | 0,5 | s |
| CLARITHROMYCIN (CH) (0,016-256) | - | 8 | 0,047 | 0,047 | s |
| AMOXICILLIN/ CLAVULANATE (XL) (0,016-256) | - | 16/8 | 0,5 | 0,38 | s |

$MIC_{bp}{}^{a}$: microbiological breakpoints for obligate homofermentative species of *Lactobacillus,* in accordance with the FEEDAP Panel Report (European Commission, 2005).
$MIC_{bp}{}^{b}$: microbiological breakpoints in accordance with Danielsen and Wind (2003).
s/r: sensitive/resistant
- : no data
?: not indicated since no breakpoint value has been suggested for this antibiotic

**Table 6.** Results of MIC determination by Etest® (AB Biodisk, Sweden) for *Str. thermophilus* Selur 12 (results of two tests are presented; 1.parall., 2. parall.).

| *Streptococcus thermophilus* Selur 12 | $MIC_{bp}{}^{a}$ | NCCLS[b] for streptococci | MIC (μg/ml) | | s/r |
|---|---|---|---|---|---|
| | | | 1.parall. | 2. parall. | |
| STREPTOMYCIN (SM) (0,064-1024) | 16 | - | 64 | 64 | r |
| GENTAMICIN (GM) (0,064-1024) | 8 | - | 16 | 24 | r |
| TETRACYCLINE (TC) (0,016-256) | 4 | - | 0,75 | 0,75 | s |
| AMPYCILLIN (AM) (0,016-256) | 4 | - | 0,125 | 0,19 | s |
| METRONIDAZOLE (MZ) (0,016-256) | - | - | > 256 | > 256 | ? |

(continued)

| *Streptococcus thermophilus* Selur 12 | $MIC_{bp}{}^{a}$ | NCCLS[b] for streptococci | MIC (μg/ml) | | s/r |
|---|---|---|---|---|---|
| | | | 1.parall. | 2. parall. | |
| CLINDAMYCIN (CM) (0,016-256) | 4 | - | 0,064 | 0,047 | s |
| CHLORAMPHENICOL (CL) (0,016-256) | 8 | - | 2 | 2 | s |
| ERYTHROMYCIN (EM) (0,016-256) | 4 | - | 6 | 6 | r |
| RIFAMPICYN (RI) (0,016-256) | - | 4 | 0,125 | 0,125 | s |
| VANCOMYCIN (VA) (0,016-256) | 4 | - | 2 | 2 | s |
| CEFOTAXIME (CTL) (0,02-32) | - | 64 | 0,19 | 0,19 | s |
| CEFACLOR (CF) (0,016-256) | - | 32 | 1,5 | 1,5 | s |
| AMOXICILLIN (AC) (0,016-256) | - | - | 0,38 | 0,38 | ? |
| AZITHROMYCIN (AZ) (0,016-256) | - | 8 | 32 | 24 | r |
| CLARITHROMYCIN (CH) (0,016-256) | - | 8 | 6 | 6 | s |
| AMOXICILLIN/ CLAVULANATE (XL) (0,016-256) | - | 16/8 | 0,19 | 0,125 | s |

$MIC_{bp}{}^{a}$: microbiological breakpoints for obligate homofermentative species of *Lactobacillus,* in accordance with the FEEDAP Panel Report (European Commission, 2005).
$MIC_{bp}{}^{b}$: microbiological breakpoints in accordance with Danielsen and Wind (2003).
s/r: sensitive/resistant
- : no data
?: not indicated since no breakpoint value has been suggested for this antibiotic

**Example 4: Antibacterial activity of novel strains**

All together antibacterial activity was tested against 31 indicator strains including different lactobacilli, *Clostridium perfringens* 1b 1106, *Cl. tyrobutyricum* DSM 2637, *Bacilus cereus* CCM 2010T, three strains of *Staphyilococcus aureus, Enterococcus durans* CCM 5612, two strains of *Enterococcus faecalis, E. faecium* LMG 11423, *Listeria monocytogenes* IM 372, *L. innocua* ATCC 33090 and E. *coli* O8:K88.

The deferred agar spot test was performed, using MRS agar with reduced concentration of glucose (0,2 % wt/v) for *Lactobacillus* strains and M17 agar for *Str. thermophilus* Selur 12 strain and 24 hours incubation of spots of test bacteria cultures before overlaying of the plates with indicator microorganisms. The overlay agar was prepared from 5 ml of appropriate soft agar for different indicator strains (MRS for lactobacilli, BHI for *B. cereus, E. coli, S. aureus* and *Listeria* strains, RCM for *Clostridium* strains and M17 for enterococci) and 50 μl of 18-h indicator cultures. Further 24-h incubation of overlaid plates was done under the conditions appropriate for individual indicator strains (30 °C/aerobiosis for *B. cereus* and *Lb. sakei,* 37 °C/aerobiosis for lactobacilli, enterococci, E. *coli, S. aureus* and *Listeria* strains, 37 °C/ anaerobiosis for clostridia).

Only the results for the indicator strains inhibited by at least one tested strains are presented in Table 8. Fig. 6 shows antimicrobial activity of *Lb. bulgaricus* strain Selur 19 and *Lb. gasseri* K7 strain against *Staphylococcus aureus* ATCC 29213.

**Table 7.** Antimicrobial activity of novel strains (*Lb. bulgaricus* Selur 19, *Str. thermophilus* Selur 12) and *Lb. gasseri* K7 strain

| Tested strains / Indicator strains | K7[1] | Selur6[1] | Selur19[1] | Selur12[2] |
|---|---|---|---|---|
| *Lb. helveticus* ATCC 15009 | ++ | + | + | + (s) |
| *Lb. gasseri* DSM 20243 | + | + | + | + |
| *Lb. sakei* NCDO 2714 | +++ (s) | + | ++ | + (s) |
| *Lb. acidophilus* ATCC4356 | +++ | ++ (s) | +++ (s) | ++ (s) |
| *Lb. johnsonii* VPI 11088 | + | + | ++ | + (s) |
| *Lb. bulgaricus* LMG 6901[T] | +++ | ++ | ++ | ++ (s) |
| *Lb. delbrueckii* LMG 6412[T] | +++ (s) | + | ++ | ++ |
| *Lb. lactis* IM 350 | +++ | + | ++ | + (s) |
| *Ec. faecalis* LMG 7937 | ++ (s) | ++ | ++ (s) | + |
| *Ec. faecalis* CCM 4647 | ++ | - | + | n.d. |
| *Ec. faecalis* LMG 11423 | + (s) | - | + (s) | n.d. |
| *Ec. faecium* LMG 11423 | +++ | ++ | ++ | + |
| *B. cereus* CCM 2010[T] | + (s) | - | + (s) | n.d. |
| *S. aureus* ATCC 29213 | +++ | +++ | +++ | - |
| *S. aureus* IM 388 | +++ | +++ | +++ | - |
| *S. aureus* IM 390 | +++ | +++ (s) | +++ | - |
| *E. coli* ATCC 25922 | - | + | ++ | n.d. |
| *E. coli* ATCC 11229 | - | - | + | n.d. |
| *Li. monocytogenes* IM 372 | +++ | +++ | +++ | + |
| *Li. innocua* ATCC 33090 | +++ | +++ | +++ | + |

Key:
[1]: spot test was performed on MRS agar plate
[2]: spot test was performed on M17 agar plate
-: no zone of inhibition was observed
+: zone of inhibition ranging between 0 and 2 mm
++: zone of inhibition ranging between 2,5 and 5mm
+++: zone of inhibition > 5,5 mm
s: sharp edge zone which is usually caused not only by acid but also other antimicrobial substances
n.d.: not determined

**Example 5: Hydrophobic properties of novel strain**

Hydrophobicity was determined according to Vinderola and Reinheimer (2003) by measurement of partition of bacterial cells between organic (n-hexadecane) and aqueous phases. Cell-surface hydrophobicity (%H) was then calculated as follows:

$$\%H = (A_0 - A)/A_0 * 100$$

where $A_0$ and A are the optical densities before and after extraction with the organic solvent, respectively. In addition to *Lb. bulgaricus* Selur 19 strains *Lb. bulgaricus* L7, L8 and L12 strains isolated from traditional Bulgarian yoghurts and *Lb. gasseri* K7 were included in testing for comparison.

High cell-surface hydrophobicity was determined for *Lb. gasseri* K7 and for *Lb. bulgaricus* Selur 19. The hydrophobicity of all other strains was quite low and ranged from 12.8 and 16.9 % (Table 9).

**Table 8.** Hydrophobicity of novel *Lb. bulgaricus* and *Str. thermophilus* strains

| Vzorec | A0 | A | H (%) |
|---|---|---|---|
| **L7** | 1,002 | 0,857 | 14,47 |
| **L8** | 1,062 | 0,883 | 16,85 |
| **L12** | 1,069 | 0,932 | 12,82 |
| **Selur 19** | 1,023 | 0,221 | 78,40 |
| **K7** | 1,058 | 0,115 | 86,13 |
| **Selur 12** | 1,007 | 0,875 | 13,11 |

**Example 6: β-galactosidase activity of strains**

ß-galactosidase (ß-Gal) activity of novel strains was evaluated by the protocol of Vinderola and Reinheimer (2003) using synthetic compound *o*-nitrophenyl-ß-D-galactopyranoside (ONPG). Briefly, ß-Gal function in the cell is to cleave lactose to glucose and galactose so that they can be used as carbon/energy sources. The synthetic compound OPNG is also recognised as a substrate which after being cleaved yields galactose and *o*-nitrophenol which has a yellow colour. When OPNG is in excess over the enzyme in a reaction, the production of o-nitrophenol per unit time is proportional to the concentration of ß-galactosidase; thus the production of yellow colour can be used to determine enzyme concentration. Absorbance values at both 420 and 560 nm were recorded for each sample and ß-Gal activity was calculated (in Miller units = standardised amount of ß-Gal activity) as follows:

$$1000 \times \frac{(A_{420} - (1.75 \times A2_{560}))}{(t \times v \times A1_{560})} = \textit{Miller units}$$

*t* = reaction time in minutes
v = volume of culture assayed in milliliters
$A_{420}$ = absorbance of yellow o-nitrophenol
$A2_{560}$ = absorbance value of the reaction mixture (the scatter from cell debris, which, when multiplied by 1,75 approximates the scatter observed at 420 nm)
$Al_{560}$ = reflects cell density in the washed cell suspension before assay

For the comparison three well known probiotic strains were included in test (*Lb. rhamnosus* ATCC 53103, *Lb. casei* DN-11401 and *Lb. casei* Shirota) (Table 10).

**Table 9.** ß- galactosidase activity of novel strain

| Strain | Miller units |
|---|---|
| *Str. thermophilus* Selur 12 | 156,2 ± 3,5 |
| *Lb. bulgaricus* Selur 19 | 3158,6 ± 17,2 |
| *Lb. gasseri* K7 | 9,4 ± 2,3 |
| *Lb. rhamnosus* ATCC 53103 | 5,6 ± 0,8 |
| *Lb. casei* DN-11401 | 8,1 ± 2,1 |
| *Lb casei* Shirota | 37,9 ± 1,2 |

**Example 7: Adhesion ability of novel strains**

An important functional attribute for probiotic bacteria is the ability of the strain to adhere to epithelial cells. In our experiments Caco-2 cells were used as *in vitro* model. Adhesion studies comprise the following steps:

a) The Caco-2 human colon adenocarcinoma cell line (IZSBS BS TCL 87) were routinely cultured in Dulbecco's modified Eagle's medium (DMEM) (Sigma, USA) with L-glutamine, and 10 % (volume fraction) of fetal calf serum Fetalclone II (Hyclone, Germany) and 50 $\mu$g/mL of gentamycin sulphate (Sigma, USA). The incubation was carried out at 37 °C in 10 % $CO_2$ atmosphere.

b) For adhesion assay, the Caco-2 cells were seeded at a concentration of $10^4$ cells/well in 24-well standard tissue culture plates. The cells were maintained for 2 weeks after the confluence, when they were considered to be fully differentiated. At least 24 h before the tests, the DMEM medium with gentamycin was replaced with the same medium without antibiotic. Lactobacilli and streptococci cells from 18-h MRS cultures were obtained by centrifugation (3500 g/ 10 min) and washed once with PBS buffer (pH=7.3) and once with the buffer used in the assay (DMEM without FBS and antibiotic, pH=7). The cell density was adjusted to the optical density of 0.5 by measuring the absorbance at 600 nm. The bacterial cells were precipitated by centrifugation and resuspended in half volume of DMEM pH=7. The exact number of viable bacterial cells used in the assays was determined for each experiment by plate counting on MRS (lactobacilli) or M17 (streptococci) agar.

c) The adhesion assays were carried out in 24-well standard tissue culture plates. After washing the Caco-2 monolayer twice with PBS (pH=7.3), 0.5 mL of lactobacilli or streptococci suspension in DMEM (containing the cells from 1 ml of the suspension of lactobacilli with OD=0,5) was added to each well and incubated for 30 min in atmosphere with 10 % $CO_2$, at 37 °C. Afterwards, the unattached bacterial cells were removed by 3-fold washing with PBS. In order to enumerate the attached bacteria, each well was treated with I mL 0.05 % Triton X-100 for 10 min. Mixtures of lysed Caco-2 cells and bacterial cells were plated on MRS agar (Merck, Germany). The enumeration was done after 72 h incubation at 37°C in microaerophilic atmosphere. Each bacterial strain was tested in ten wells. Results are the average value of 10 data and are presented in Fig. 7.

*Lb. bulgaricus* Selur 19 was tested also for competition and exclusion properties against two strains, *S. aureus* ATCC 29213 and *E. coli* ATCC 25922. The latter two strains were chosen on the basis of results of deferred agar spot assays - they were inhibited by *Lb. bulgaricus* Selur 19. *S. aureus* ATCC 29213 and *E. coli* ATCC 25922 were cultured in BHI broth and prepared as described for lactobacilli, with only difference that 10-fold more diluted cell suspensions those of lactobacilli were applied in the test. (Suspension was adjusted to OD=0,5, further diluted 1:10, centrifuged; then the cells were resuspended in appropriate amount of DMEM).

In competition assay *(Lb. + S. aureus; Lb. + E. coli),* lactobacilli were added to Caco-2 cells in the wells simultaneously with *S. aureus* and *E. coli.* To test possible exclusion of S. *aureus* and *E. coli* by lactobacilli *(Lb. /S. aureus; Lb. / E. coli),* the incubation of Caco-2 cells with lactobacilli was followed by washing of unbound cells, addition of S. *aureus* or *E. coli* and another 30 min of incubation. Caco-2 cells were lysed by 0.05 % Triton X-100 (Sigma Chemical Co., St. Louis, MO, USA) for 5 min, and adhered *S. aureus* and *E. coli* were enumerated (cfu/well) by plate counting on BHI and VRB agar media (Merck), respectively. BHI and VRB plates were incubated for 24 h at 30°C.

The adhesion of *Lb. bulgaricus* Selur 19 and *Str. thermophilus* Selur 12 strains was in general at least comparable or in the case when % of adhesion was calculated (adhered cfu/well divided with added cfu/well expressed in %) even better from that determined for *Lb. gasseri* K7 and some other probiotic strains (*Lb. rhamnosus* GG, *Lb. johnsonii* Lj1, *Lb. reuteri* ING 1, *Lb.* Shirota SHI) (Fig. 7).

When competition between *E. coli* ATCC 25922 and *Lb. bulgaricus* Selur 19 or between *S. aureus* ATCC 29213 and *Lb. bulgaricus* Selur 19 for adhesion to Caco-2 cells was tested 1.75 x $10^7$ cfu/well of Selur 19 and 2.22 x $10^7$ cfu/well of E. *coli* (ratio of added cfu *E. coli:* Selur 19 = 1,3 : 1) or 1.75 x $10^7$ cfu/well of Selur 19 and 0.52 x $10^7$ cfu/well of *S. aureus* (ratio of added cfu *S. aureus:* Selur 19 = 0,3 : 1) was added.

*Lb. bulgaricus* Selur 19 was able to inhibit the adhesion of *E. coli* ATCC 25922 when both strains were added to Caco-2 cells simultaneously, at similar concentrations. In the exclusion assay (Selur 19 / *E. coli)* the adhesion of *E. coli* was not inhibited. However, Selur 19 was not able to inhibit the adhesion of *S. aureus* ATCC 29213, although the concentration of lactobacilli was about 3-times higher (Table 11).

**Table 10.** Effect of *Lb. bulgaricus* Selur 19 on the adhesion ability of *E.coli* ATCC 25922 and *S. aureus* ATCC 29213

| Assay | Adhered *E. coli* (log cfu/well) | Adhesion inhibition (%)* |
|---|---|---|
| *E. coli* | 6,21±0.08 | |
| Competition (*E. coli* + Selur 19) | 5,72±0.08 | 67 |
| Exclusion (Selur 19/*E. coli)* | >7,3 | 0 ** |
| | Adhered *S. aureus* (log cfu/well) | |
| *S. aureus* | 5,39±0.10 | |
| Competition (*S. aureus* + Selur 19) | 5,39±0.11 | 0 |
| Exclusion (Selur 19/*S. aureus)* | 5,63±0.13 | 0 ** |
| * Adhesion inhibition (%) was calculated by comparison of results (adhered cfu of *E.coli*/well) obtained in assays E. *coli* + Selur 19 and Selur 19/*E. coli* with adhesion ofE. *coli* alone (100%).<br>** Adhesion even better as when alone. | | |

**Example 8: Immune response of THP-1 macrophage cell line on the *in vitro* stimulation with and K7**

and K7 were tested for their ability to affect the secretion of cytokines by **THP-1** macrophage cell line. Due to their well-known probiotic effects including modulation of immune response, *Lb. rhamnosus* GG and Lb. casei DN-114001 were used as positive controls.

Procedure was as follows, briefly:

The THP-1 cell line was stimulated with TPA (phorbol ester) for 24 hours to induce the differentiation into mature macrophages. Differentiated cells were washed and incubated for 24 hours with fresh medium (RPMI-1640) without TPA, supplemented with 10% bovine serum and antibiotics. The experiment was performed on the third day when lactobacilli, LPS (lipopolysaccharides of E. *coli* O111:B4) were added and incubated with macrophages for 24 hours. Concentration of six different cytokines (IL-8, IL-1β, IL-6, IL-10, TNF in IL-12p70) was measured by BD™ CBA (Cytometric Bead Array) human inflammation kit, using flow cytometer (BD FACSCalibur).
and K7 strains did not induce secretion of inflammatory cytokines (IL-8, IL-6 and TNF) by macrophage cell line THP-1, while their secretion was induced by the addition of LPS. When LPS and or K7 strains were applied simultaneously, both strains reduced the concentration of the majority of inflammatory cytokines. Most prominent changes in cytokine concentrations were noted in the levels of IL-8 (Table 12). When or K7 were added alone we noted a slight increase in IL-1β (mediator of local inflammation) concentrations, however the effect was not statistically significant. All immune responses were comparable to control strains. The concentrations of IL-10 and IL-12p70 were not affected by either of the strains tested.

**Table 11.** Reduction of IL-8 production induced by LPS

| Bacterial strain | Reduction % |
|---|---|
| *Lb. gasseri* K7 | 93 |
| *Lb. crispatus* 4/26* | 86 |
| *Lb. bulgaricus* | 99 |
| *Lb. rhamnosus* GG | 95 |
| *Lb. casei* DN 114001 | 99 |
| * strain isolated from pig's intestine | |

**Example 9: Fermentative ability of different combinations of strains**

Different combinations of strains and fermentation temperatures were tested. Pure overnight cultures were combined and inoculated in sterile skimmed milk at a level of 2 % and incubated at 42 °C and 37 °C (only combinations S20 + L89 and S20 + L89 + K7). During the fermentation pH was measured and fermentation was stopped when pH of 4,6 was reached. After fermentation samples of fermented milk were plated on MRS, M17 and MRS with clindamycin (0,1 mg/l) agar media for selective enumeration of streptococci, lactobacilli and *Lb. gasseri* K7.

Changes of pH values during fermentation of milk inoculated with *Str. thermophilus* Selur 12 and different lactobacilli strains (Selur 19 and Selur 19 + K7) at a ratio 1:1 or 1:1:1 (Selur 12 + Selur 19 + K7) at 42 °C and 37 °C (only Selur 12 + Selur 19 and Selur 12 + Selur 19 + K7) are presented in Fig. 8. The average number (in cfu/mL) of single strains after fermentations/in fermented milk is presented in Table 13.

**Table 12.** Average number of *Str. thermophilus* Selur 12, *Lb. bulgaricus* or Selur 19 and *Lb. gasseri* K7 in fermented milk samples (cfu/mL)

| Combination | *Str. thermophilus* | *Lb. delbrueckii* ssp. *bulgaricus* | *Lb. gasseri* K7 |
|---|---|---|---|
| Selur 12 + | $2,16*10^8$ | $9,30*10^8$ | |
| Selur 12 + Selur 19 | $3,10*10^9$ | $8,27*10^8$ | |
| Selur 12 + Selur 19 + K7 | $3,90*10^9$ | $1,24*10^9$ | $8,70*10^8$ |
| Selur 12 + Selur 19* | $8,20*10^9$ | $4,20*10^9$ | |
| Selur 12 + Selur 19 + K7* | $1,57*10^9$ | $1,47*10^9$ | $1,57*10^9$ |
| * fermentation at 37 °C | | | |

**Example 10: Technology for production of biomass and preparation of probiotic starter cultures, food or feed supplements and pharmaceutical preparations**

Strains are prepared in pure overnight (18-hours) cultures (growth conditions are described in Table 14). Overnight broth cultures are centrifuged (10 min, 6000 g), supernatant removed and cells re-suspended in 10-times lower quantity of Ringer solution. Cell concentration in 1 ml of cell suspension is about $1x10^9$- $1x10^{10}$ cells per ml. Cell suspensions of Selur 12, and Selur 19 are inoculated separately or in combinations into sterilised reconstituted skim milk (1 % inoculum), and *Lb. gasseri* K7 (1 % inoculum) into reconstituted skim milk supplemented with 0,4 % of yeast extract (Biolife 412220; addition into milk before milk sterilisation), respectively for preparation of mother cultures which can be used as feeder cultures for fermentation in bioreactor. Overnight cultivation is carrying at 42 °C for Selur 12, and Selur 19 and at 37 °C for *Lb. gasseri* K7.

Biomass is produced by fermentation of milk, whey or vegetable substrates such as soya, rice or other grain milk. The steps in procedure where milk is used as substrate are as follow:

a) For medium preparation 100 g/L of skimmed milk powder and 4 g/L of yeast extract are dissolved in tap water and mixture is sterilized at 115 ° for 30 min in bioreactor. After sterilization medium is cooled to temperature appropriate for different strain or combination of strains.

b) Biomass production by fermentation - technological parameters

<u>Example</u> 1

Prepared medium in bioreactor is inoculated with I % of fresh 18-hours culture of *Str. thermophilus* Selur 12 and 1 % of fresh 18-hours culture of *Lb. bulgaricus* Selur 19. Fermentation/cultivation is carrying through 1 h at 42 °C than the temperature is raised to 44 °C and during the fermentation for the following 3-4 hours three neutralizations with $NH_4OH$ are done: I - when pH drops to 5,0 it is raised to pH 5,7; II - when pH drops to 4,9 it is raised to pH 5,7; III - when pH drops to 4,8 it is raised to pH 5,7. Agitation of fermenting broth is performed only during neutralization. At the end of fermentation the 10 % (v/v) of cryo-protector (200 g/L solution of sterile skimmed milk powder) is added and fermentation broth in bioreactor cooled down to 22 °C for 30 min. The whole fermentation broth is lyophilized. Before lyophilization the pH of broth is rised to 6,2.

<u>Example 2</u>

Prepared medium in bioreactor is inoculated with 2 % of fresh 18-hours culture of *Lb. gasseri* K7 and 16-hours fermentation at 37 °C is performed without pH regulation. At the end of fermentation the 10 % (v/v) of cryo-protector (200 g/L solution of sterile skimmed milk powder) is added and fermentation broth in bioreactor cooled down to 22 °C for 30 min. The whole fermentation broth is lyophilized. Before lyophilization the pH of broth is rised to 6,2.

Example 3

Prepared medium in bioreactor is inoculated with 2 % of fresh 18-hours culture of *Lb. gasseri* K7 and fermentation at 37 °C is performed without pH regulation. After 16-hours of fermentation/cultivation at 37 °C pH is raised to 5,7 and fermentation broth inoculated with 2 % of fresh 18-hours culture of *Lb. bulgaricus* Selur 19. After inoculation the temperature is raised to 44 °C and during the fermentation for the following 3-4 hours neutralization with $NH_4OH$ from pH 5,0 to pH 5,7 is performed three times. Agitation of fermenting broth is performed only during neutralization. At the end of fermentation the 10 % (v/v) of cryo-protector (200 g/L solution of sterile skimmed milk powder) is added and fermentation broth in bioreactor cooled down to 22 °C for 30 min. Before lyophilization the pH of broth is rised to 6,2.

c) Lyophilization - technological parameters

Thickness of liquid layer - 10 mm; cooling at 4 °C for 3 hours; freezing at -40 °C for 90 minutes; heating at 35 °C until the end of evaporation (<6 % of water).

The preparations comprise the freeze-dried bacteria in concentration of viable cells, which is usually in the range of $10^7$ to $10^{10}$ cfu (colony forming units) per gram, less than 400 mg of lactose per gram and lactulose which is in the range of 25 to 50 mg/g.

Table 13. Optimal growth conditions for strains

| **Strain** | **Growth medium** | **Temperature/condition** |
|---|---|---|
| *Str. thermophilus* Selur 12 | M 17 (broth, agar) or 10 % reconstituted skim milk | 42 °C / aerobically |
| *Lb. bulgaricus* Selur 19 | MRS (broth, agar) or 10 % reconstituted skim milk | 42 °C / anaerobically |
| *Lb. gasseri* K7 | MRS (broth, agar) | 37 °C / anaerobically |

**Example of a product (pharmaceutical composition):**

| ***Liolact, 400mg capsules*** | | |
|---|---|---|
| Contents | For one capsule | |
| | mg | % |
| Lyophilizate *Lactobacillus gasseri K7* | 160 | 39,41% |
| Lyophilizate *Lactobacillus bulgaricus* BF + *Streptococcus thermophilus* Selur 12 | 80 | 19,70% |
| Lyophilizate *Lactobacillus bulgaricus* | 160,0 | 39,41% |
| $SiO_2$ (Aerosil A200) | 2,0 | 0,49% |
| Magnesium stearate | 4,0 | 0,99% |
| Total for I capsule | 406,0 | 100% |
| 1. The required amounts of each component are extended, as a percent of total mixture amount, required to produce the batch.<br>2. All components are filtered through a filter with pore size of 0.5 mm:<br>3. Mixed:<br>3.1. Feeding to the mixer for 30-min mixing, but for the magnesium stearate;<br>3.2. Adding to the mixture from point 3.2. the preliminary filtered amount of magnesium stearate and mixing for another 10 min.<br>4. The ready-made mixture packed in containers. | | |

**REFERENCES:**

1. Basic Local Alignment Search Tool: http://www.ncbi.nlm.nih.gov/blast/

2. Bogovič Matijašić B, Rogelj I. *Lactobacillus* K7 - A New Potential Probiotic Strain. *Food Technol. Biotechnol.* 2000; 38: 113-119.

3. Bogovič Matijašić B, Narat M, Zorič M. Adhesion of two *Lactobacillus gasseri* Probiotic Strains on Caco-2 Cells. *Food Technol. Biotechnol.* 2003; 41: 83-88.

4. Bogovič Matijašić B, Stojković S, Salobir J, Malovrh S, Rogelj I. Evaluation of the *Lactobacillus gasseri* K7 and LF221 strains in weaned piglets for their possible probiotic use and their detection in the faeces. *Anim. Res.* 2004; 53: 35-44.

5. Bogovič Matijašić B, Narat M, Zorič Peternel M, Rogelj I. Ability of *Lactobacillus gasseri* K7 to inhibit *Escherichia coli* adhesion in vitro on Caco-2 cells and ex vivo on pigs'jejunal tissue. *Int. J. Food Microbiol.* 2006; 107: 92 - 96.

6. Bogovič Matijašić B, Koman Rajšp M, Perko B, Rogelj I. Inhibition of *Clostridium tyrobutyricum* in cheese by *Lactobacillus gasseri. Int. Dairy J.* 2007; 17 (2): 157-166.
7. Chopra A, Doiphode VV. Ayurvedic medicine - Core concept, therapeutic principles, and current relevance. Med. Clin.North Am. 2002; 86 (1): 75.

8. Čanžek Majhenič A, Bogovič Matijašić B, Rogelj I. Chromosomal location of the genetic determinants for bacteriocins produced by *Lactobacillus gasseri* K7. *J. Dairy Res.* 2003; 70: 199-203.
9. Danielsen M, Wind A. Susceptibility of Lactobacillus spp. to antimicrobial agents. Int. J. Food Microbiol. 2003; 82: 1-11.
10. Delgado S, O'Sullivan E, Fitzgerald G, Mayo B. Subtractive Screening for Probiotic Properties of Lactobacillus Species from the Human Gastrointestinal Tract in the Search for New Probiotics. J. Food Sci. 2007; 65 (8): M310-M315.
11. Elli M, Callegari ML, Ferrari S, Bessi E, Cattivelli D, Soldi S, Morelli L, Feuillerat NG, Antoine JM. Survival of Yogurt Bacteria in the Human Gut. Appl. Environ. Microbiol. 2006; 72 (7): 5113-5117.
12. European Commission, 2005. European Commission, Opinion of the FEEDAP Panel on the updating of the criteria used in the assessment of bacteria for resistance to antibiotics of human or veterinary importance, EFSAJ. 2005; 223: 1-12.
13. FAO/WHO, 2002. Guidelines for the evaluation of probiotics in food. London, Ontario: Food and Agriculture Organization of United Nations and World Health Organization Working Group Report.
14. Final Technical report for FSA project ref G01022. An evaluation of probiotic effects in the human gut: microbial aspect, FSA 2005, ref G01022, 2-22.
15. García-Albiach R, José M, de Felipe P, Angulo S, Morosini M-I, Bravo D, Baquero F, del Campo R. Molecular analysis of yogurt containing Lactobacillus delbrueckii subsp. bulgaricus and Streptococcus thermophilus in human intestinal microbiota Am. J. Clin. Nutr. 2008; 87: 91-96.
16. Guarner F, Perdigon G, Corthier G, Salminen S, Koletzko B, Morelli L. Should yoghurt cultures be considered probiotic? Br. J. Nutr. 2005; 93: 783-786.
17. Klare I, Konstabel C, Müller-Bertling S, Reissbrodt R, Huys G, Vancanneyt M, Swings J, Goossens H, Witte W. Evaluation of New Broth Media for Microdilution Antibiotic Susceptibility Testing of Lactobacilli, Pediococci, Lactococci, and Bifidobacteria. Appl. Environ. Microbiol. 2005; 71 (12): 8982-8986.
18. Klijn N, Weerkamp AH, De Vos WM. Identification of Mesophilic Lactic Acid Bacteria by Using Polymerase Chain Reaction-Amplified Variable Regions of 16S rRNA and Specific DNA Probes. Appl. Environ. Microbiol. 1991; 57 (11): 3390-3393.
19. Korhonen H, Pihlanto A. Bioactive peptides: Production and functionality. Int. Dairy J. 2006; 16: 945-960.
20. Kullen MJ, Sanozky-Dawes RB, Crowell DC, Klaenhammer TR. Use of the DNA sequence of variable regions of the 16S rRNA gene for rapid and accurate identification of bacteria in the Lactobacillus acidophilus complex. J. Appl. Microbiol. 2000; 89 (3): 511-516.
21. Mater DD, Bretigny L, Firmesse O, Flores MJ, Mogenet A, Bresson JL, Corthier G. Streptococcus thermophilus and Lactobacillus delbrueckii subsp. bulgaricus survive gastrointestinal transit of healthy volunteers consuming yogurt. FEMS Microbiol Lett. 2005; 15 (2): 185-7.
22. Meisel H. Overview on Milk Protein-derived Peptides. Int. Dairy J, 1998; 8 (5/6): 363-373.
23. Mitsuoka T. Significance of dietary modification of intestinal flora and intestinal environment. Biosci. Microflora, 2000; 19: 15-25.

24. Rogelj I, Bogovič Matijač ić B. Lactobacillus gasseri LF221 and K7 - from isolation to application. Biologia, 2006; 61: 761-769.

25. Rossetti L, Giraffa G. Rapid Identification of Dairy Lactic Acid Bacteria by M13-generated RAPD-PCR Fingerprint Databases. J.Microbiol. Meth. 2005; 63: 135-144.

26. Shah NP. Functional cultures and health benefits. Int. Dairy J. 2007; 17: 1262-1277.

27. Smithers GW. Whey and whey proteins-From 'gutter-to-gold'. Int. Dairy J. 2008; 18: 695-704.

28. Tannock GW, Tilsala-Timisjarvi A, Rodtong S, Ng J, Munro K, Alatossava T. Identification of Lactobacillus Isolates from the Gastrointestinal Tract, Silage, and Yoghurt by 16S-23S rRNA Gene Intergenic Spacer Region Sequence Comparisons. Appl. Environ. Microbiol. 1999; 65 (9): 4264-4267.

29. Tilsala-Timisjärvi A, Alatossava T. Development of Oligonucleotide Primers from the 16S-23S rRNA Intergenic Sequences for Identifying Different Dairy and Probiotic Lactic Acid Bacteria by PCR. Int. J. Food Microbiol. 1997; 35: 49-56.

30. Torriani S, Zapparoli G, Dellaglio F. Use of PCR-Based Methods for Rapid Differentiation of Lactobacillus delbrueckii subsp. bulgaricus and L. delbrueckii subsp. lactis. Appl. Environ. Microbiol. 1999; 65 (10): 4351-4356.

31. Tynkkynen S, Satokari R, Saarela M, Mattila-Sandholm T, Saxelin M. Comparison of ribotyping, randomly amplified polymorphic DNA analysis, and pulsed-field gel electrophoresis in typing of Lactobacillus rhamnosus and L. examplei strains. Appl. Environ. Microbiol. 1999; 65: 3908-3914.

32. Van Reenen CA, Dicks LMT. Evaluation of Numerical Analysis of Random Amplified Polymorphic DNA (RAPD)-PCR as a Method to Differentiate Lactobacillus plantarum and Lactobacillus pentosus. Cur. Microbiol. 1996; 32: 183-187.

33. Vinderola CG, Reinheimer JA. Lactic acid starter and probiotic bacteria: a comparative "in vitro" study of probiotic characteristics and biological barrier resistance. Food Res. Int. 2003; 36: 895-904.

34. Zorič Peternel M. Aktivnost bakteriocinskih kompleksov sevov Lactobacillus gasseri K7 in Lactobacillus gasseri LF221 v različ nih ekspresijskih sistemih: doktorska diserlacija = Activity of bacteriocin complex of Lactobacillus gasseri K7 and Lactobacillus gasseri LF221 strains in different expression systems: doctoral dissertation. Ljubljana, 2007: 138p.

## Claims

1. *Lactobacillus delbrueckii* ssp. *bulgaricus* bacterial strain Selur 19 isolated from traditional yoghurt produced from raw milk from the Rhodope Mountains (Bulgaria) and deposited in the Czech Collection of Microorganisms (CCM) under Registration Number CCM 7713.

2. A starter culture **characterised by** the fact that it contains the bacterial strain in accordance with Claim 1 or *Streptococcus thermophilus* bacterial strain Selur 12, deposited in the Czech Collection of Microorganisms (CCM) under Registration Number CCM 7711 jointly with *Lb. gasseri* probiotic strain K7, deposited in the Czech Collection of - Microorganisms (CCM) under Registration Number CCM 7710, where the *Lb. gasseri* strain K7 is present in concentration of viable cells, which is within the range of $10^6$ to $10^{11}$ cfu (colony forming units) per gram or ml of the composition of at least $10^6$ cfu per gram or ml of the composition, such as at least $10^7$ cfu/g or ml, i.e. at least $10^8$ cfu/g or ml, such as at least $10^9$ cfu/g or ml, or at least $10^{10}$ cfu/g or ml, such as least $10^{11}$ cfu/g or ml.

3. The use of the starter culture according to the Claim 2 in fermentation of each material which is generally subject to a fermentation stage with lactic acid bacteria such as milk, plant materials, meat products, fruit juices and dough.

4. A food product containing carrier material, *Lb. gasseri* strain K7 (CCM 7710), and the strain according to Claims 1, or *Streptococcus thermophilus* bacterial strain Selur 12 (CCM 7711), or a starter culture according to Claim 2.

**5.** A product, according to Claim 4, **characterised by** the fact that the carrier material represents food composition of milk, yoghurt, white cheese, fermented milks, fermented products based on milk, fermented products based on meat, products based on fermented grain materials, powders based on milk and wheat, baby food, ice-cream, juices, candies or chewing-gums.

**6.** Food supplement composition intended for use by humans and animals, **characterised by** the fact that it contains the bacterial strain according to Claim 1, or *Streptococcus thermophilus* bacterial strain Selur 12 (CCM 7711), jointly with the *Lb. gasseri* strain K7 (CCM 7710).

**7.** Pharmaceutical composition, **characterised by** the fact that it contains the bacterial strain according to Claims 1, or *Streptococcus thermophilus* bacterial strain Selur 12 (CCM 7711 ), jointly with *Lb. gasseri* strain K7 (CCM 7710).

**8.** A composition, according to Claims 6 or 7, **characterised by** the fact that it contains the strain in accordance with Claim 1, or *Streptococcus thermophilus* bacterial strain Selur 12 (CCM 7711) in quantities of $10^7$ cfu/g to about $10^{12}$ cfu/g of the carrier material, and *Lb. gasseri* strain K7 (CCM 7710), in quantities of about $10^8$ cfu/g to about $10^{12}$ cfu/g carrier material, preferably of $10^9$ cfu/g to about $10^{12}$ cfu/g, more preferred than of about $10^{10}$ cfu/g to about $10^{12}$ cfu/g carrier material.

**9.** A bacterial strain, a starter culture, a composition or a product in accordance with Claim 1, or Claim 2, or Claims 4 to 5 or Claims 6 to 8 respectively, intended for therapeutic or prophylactic treatment of diseases of humans or animals.

**10.** Use of a strain, a starter culture, a composition or a product in accordance with Claim 1, or Claim 2, or Claims 4 to 5 or Claims 7 to 9 respectively, to obtain a product intended for therapeutic or prophylactic treatment of human or animal diseases.

**11.** Use, according to Claim 10, **characterised by** the fact that disease was selected from the group of gastrointestinal tract disease, comprising antibiotic-associated disorders, gastroenteritis, traveller's diarrhoea, acute child diarrhoea, lactose intolerance, gastro-intestinal infections, including *Clostridium difficile* infections, irritable bowel syndrome (IBS), inflammatory bowel disease (IBD), and other immunomodulating syndromes.

**Example 1: Characterization of the novel *Lb. bulgaricus* Selur 19 and *Str. thermophilus* Selur 12 strains**

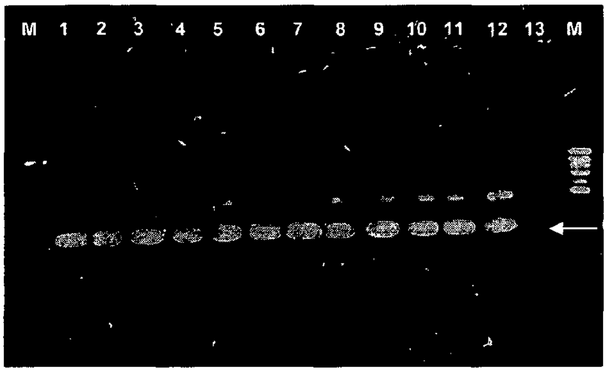


200 bp

Fig. 1.

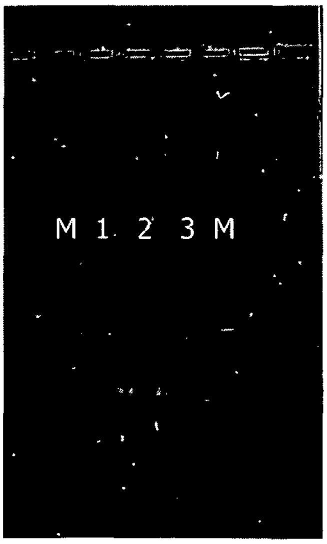


260 bp

Fig. 2.

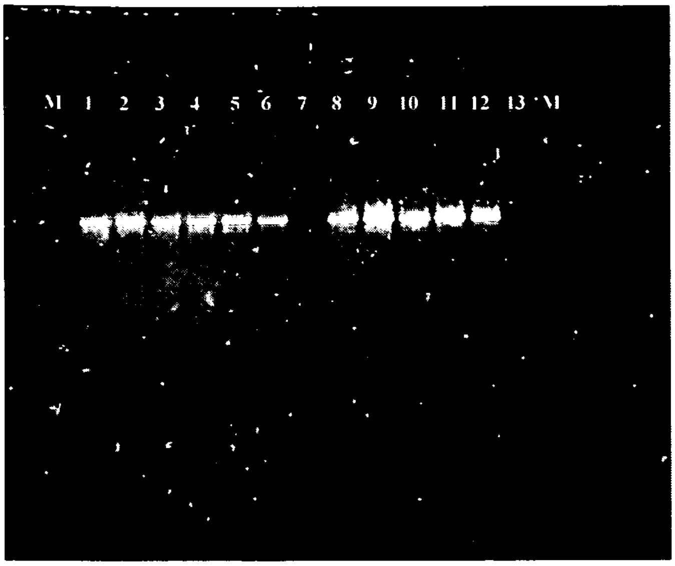


Fig. 3.

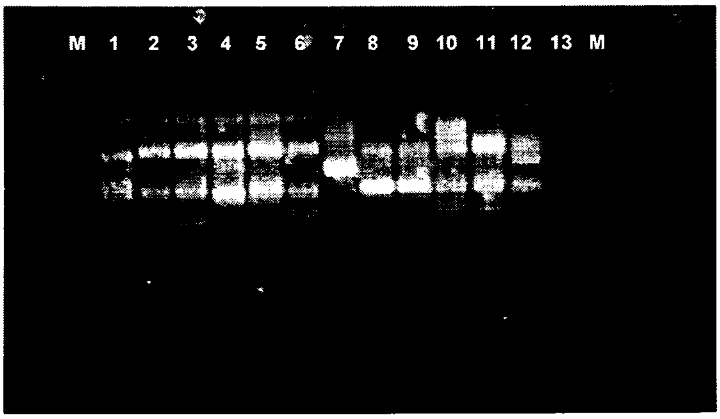


Fig. 4

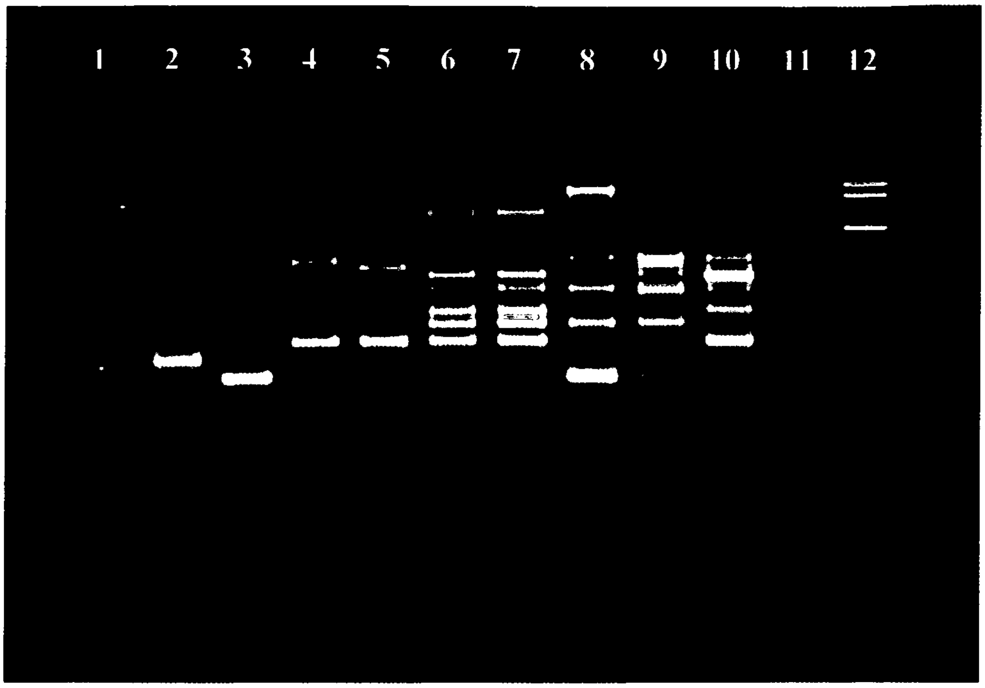


Fig. 5

**Example 4: antibacterial activity of novel strains**

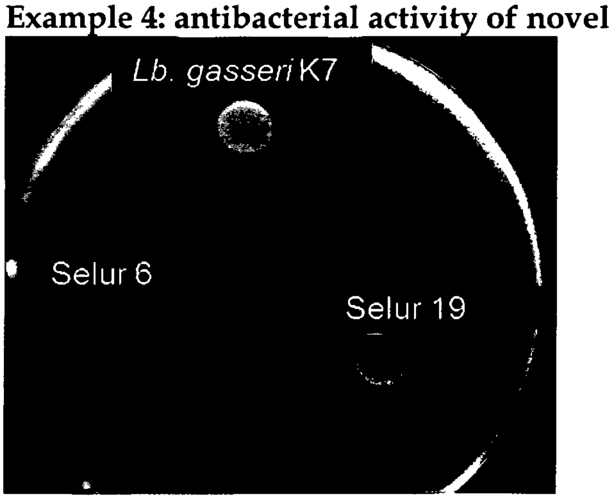


Fig. 6

### Example 7: Adhesion ability of novel strains

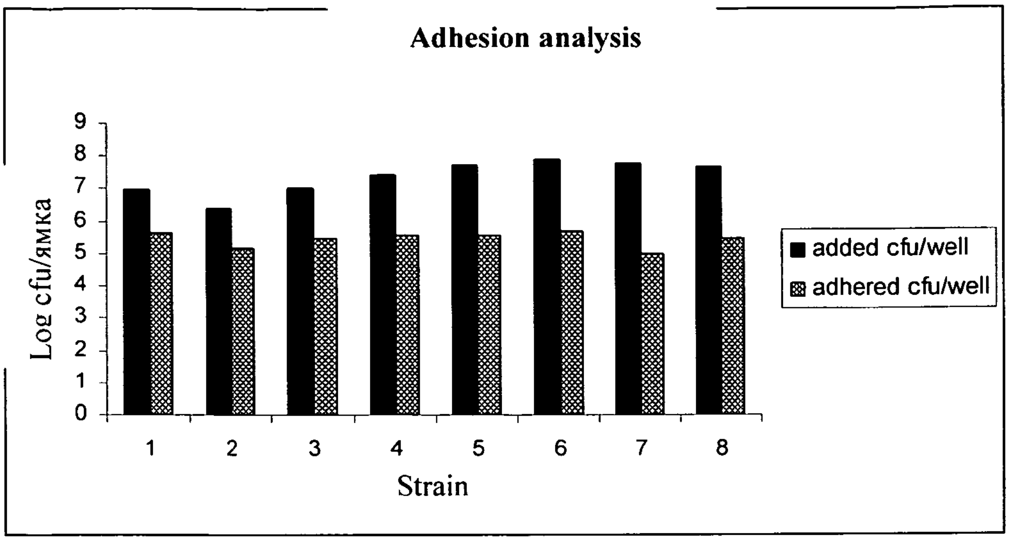


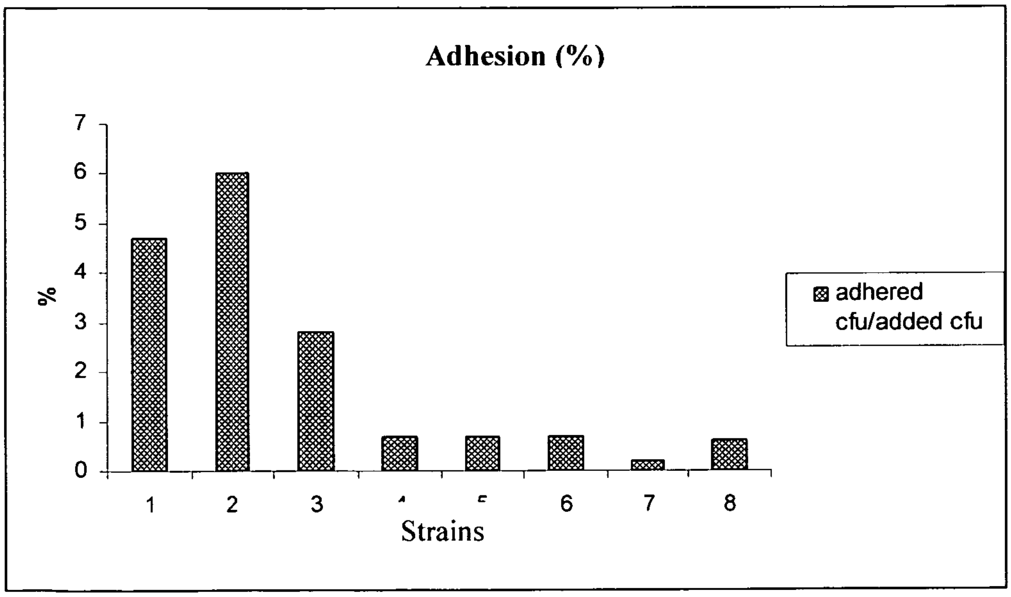


Fig. 7

Example 9: Fermentative ability of different combinations of strains

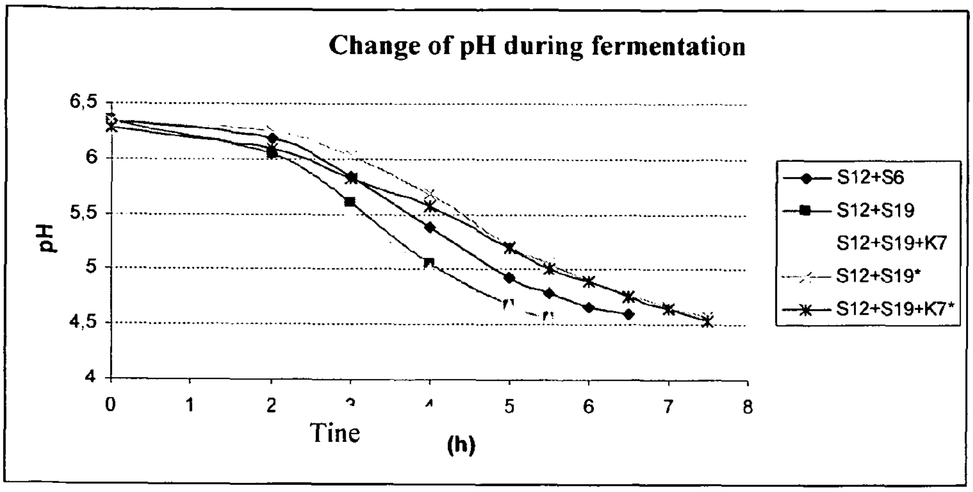


Fig. 8.

Europäisches Patentamt
European Patent Office
Office européen des brevets

# EUROPEAN SEARCH REPORT

Application Number
EP 13 00 0103

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BOLOTIN ALEXANDER ET AL: "Complete sequence and comparative genome analysis of the dairy bacterium Streptococcus thermophilus", NATURE BIOTECHNOLOGY, NATURE PUBLISHING GROUP, NEW YORK, NY, US, vol. 22, no. 12, 1 December 2004 (2004-12-01), pages 1554-1558, XP002460395, ISSN: 1087-0156, DOI: 10.1038/NBT1034 * page 1554, left-hand column - page 1557, right-hand column * ----- | 4,5,9-11 | INV. C12R1/225 A61K35/74 |

TECHNICAL FIELDS SEARCHED (IPC)
C12R

The present search report has been drawn up for all claims

2

| Place of search | Date of completion of the search | Examiner |
|---|---|---|
| Munich | 20 June 2013 | Stoyanov, Borislav |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number

EP 13 00 0103

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 13 00 0103

The Search Division considers that the present European patent application does not comply with the requirements of unity of invention and relates to several inventions or groups of inventions, namely:

1. claims: 1(completely); 2-11(partially)

    Bacterial strain Selur 19 and products containing Selur 19 alone or in combination with other strains and the uses thereof

    ---

2. claims: 2-11(partially)

    Products containing Selur 12 alone, or in combination with other strains and the uses thereof

    ---

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9620607 A
- WO 2005056028 A
- WO 2008002484 A
- JP 04320642 B
- JP 05227946 B
- US 6596530 B
- WO 2004003235 A
- WO 2008016214 A
- US 2008233104 A
- WO 8905849 A
- NZ 523010


### Non-patent literature cited in the description


- **CHOPRA A, DOIPHODE VV.** Ayurvedic medicine - Core concept, therapeutic principles, and current relevance. *Med. Clin.North Am.,* vol. 86 (1), 75
- **DANIELSEN M ; WIND A.** Susceptibility of Lactobacillus spp. to antimicrobial agents. *Int. J. Food Microbiol.,* 2003, vol. 82, 1-11
- **DELGADO S ; O'SULLIVAN E ; FITZGERALD G ; MAYO B.** Subtractive Screening for Probiotic Properties of Lactobacillus Species from the Human Gastrointestinal Tract in the Search for New Probiotics. *J. Food Sci.,* 2007, vol. 65 (8), M310-M315
- **ELLI M ; CALLEGARI ML ; FERRARI S ; BESSI E ; CATTIVELLI D ; SOLDI S ; MORELLI L ; FEUILLERAT NG ; ANTOINE JM.** Survival of Yogurt Bacteria in the Human Gut. *Appl. Environ. Microbiol.,* 2006, vol. 72 (7), 5113-5117
- European Commission, Opinion of the FEEDAP Panel on the updating of the criteria used in the assessment of bacteria for resistance to antibiotics of human or veterinary importance. *EFSAJ.,* 2005, vol. 223, 1-12
- Guidelines for the evaluation of probiotics in food. London, Ontario. FAO/WHO, 2002
- *An evaluation of probiotic effects in the human gut: microbial aspect,* 2005
- **GARCÍA-ALBIACH R ; JOSÉ M ; DE FELIPE P ; ANGULO S ; MOROSINI M-I ; BRAVO D ; BAQUERO F ; DEL CAMPO R.** Molecular analysis of yogurt containing Lactobacillus delbrueckii subsp. bulgaricus and Streptococcus thermophilus in human intestinal microbiota. *Am. J. Clin. Nutr.,* 2008, vol. 87, 91-96
- **GUARNER F ; PERDIGON G ; CORTHIER G ; SALMINEN S ; KOLETZKO B ; MORELLI L.** Should yoghurt cultures be considered probiotic?. *Br. J. Nutr.,* 2005, vol. 93, 783-786
- **KLARE I ; KONSTABEL C ; MÜLLER-BERTLING S ; REISSBRODT R ; HUYS G ; VANCANNEYT M ; SWINGS J ; GOOSSENS H ; WITTE W.** Evaluation of New Broth Media for Microdilution Antibiotic Susceptibility Testing of Lactobacilli, Pediococci, Lactococci, and Bifidobacteria. *Appl. Environ. Microbiol.,* 2005, vol. 71 (12), 8982-8986
- **KLIJN N ; WEERKAMP AH ; DE VOS WM.** Identification of Mesophilic Lactic Acid Bacteria by Using Polymerase Chain Reaction-Amplified Variable Regions of 16S rRNA and Specific DNA Probes. *Appl. Environ. Microbiol.,* 1991, vol. 57 (11), 3390-3393
- **KORHONEN H ; PIHLANTO A.** Bioactive peptides: Production and functionality. *Int. Dairy J.,* 2006, vol. 16, 945-960
- **KULLEN MJ ; SANOZKY-DAWES RB ; CROWELL DC ; KLAENHAMMER TR.** Use of the DNA sequence of variable regions of the 16S rRNA gene for rapid and accurate identification of bacteria in the Lactobacillus acidophilus complex. *J. Appl. Microbiol.,* 2000, vol. 89 (3), 511-516
- **MATER DD ; BRETIGNY L ; FIRMESSE O ; FLORES MJ ; MOGENET A ; BRESSON JL ; CORTHIER G.** Streptococcus thermophilus and Lactobacillus delbrueckii subsp. bulgaricus survive gastrointestinal transit of healthy volunteers consuming yogurt. *FEMS Microbiol Lett.,* 2005, vol. 15 (2), 185-7
- **MEISEL H.** Overview on Milk Protein-derived Peptides. *Int. Dairy J,* 1998, vol. 8 (5/6), 363-373
- **MITSUOKA T.** Significance of dietary modification of intestinal flora and intestinal environment. *Biosci. Microflora,* 2000, vol. 19, 15-25
- B. Lactobacillus gasseri LF221 and K7 - from isolation to application. *Biologia,* 2006, vol. 61, 761-769

- **ROSSETTI L ; GIRAFFA G.** Rapid Identification of Dairy Lactic Acid Bacteria by M13-generated RAPD-PCR Fingerprint Databases. *J.Microbiol. Meth.,* 2005, vol. 63, 135-144
- **SHAH NP.** Functional cultures and health benefits. *Int. Dairy J.,* 2007, vol. 17, 1262-1277
- **SMITHERS GW.** Whey and whey proteins-From 'gutter-to-gold. *Int. Dairy J.,* 2008, vol. 18, 695-704
- **TANNOCK GW ; TILSALA-TIMISJARVI A ; RODTONG S ; NG J ; MUNRO K ; ALATOSSAVA T.** Identification of Lactobacillus Isolates from the Gastrointestinal Tract, Silage, and Yoghurt by 16S-23S rRNA Gene Intergenic Spacer Region Sequence Comparisons. *Appl. Environ. Microbiol.,* 1999, vol. 65 (9), 4264-4267
- **TILSALA-TIMISJÄRVI A ; ALATOSSAVA T.** Development of Oligonucleotide Primers from the 16S-23S rRNA Intergenic Sequences for Identifying Different Dairy and Probiotic Lactic Acid Bacteria by PCR. *Int. J. Food Microbiol.,* 1997, vol. 35, 49-56
- **TORRIANI S ; ZAPPAROLI G ; DELLAGLIO F.** Use of PCR-Based Methods for Rapid Differentiation of Lactobacillus delbrueckii subsp. bulgaricus and L. delbrueckii subsp. lactis. *Appl. Environ. Microbiol.,* 1999, vol. 65 (10), 4351-4356
- **TYNKKYNEN S ; SATOKARI R ; SAARELA M ; MATTILA-SANDHOLM T ; SAXELIN M.** Comparison of ribotyping, randomly amplified polymorphic DNA analysis, and pulsed-field gel electrophoresis in typing of Lactobacillus rhamnosus and L. examplei strains. *Appl. Environ. Microbiol.,* 1999, vol. 65, 3908-3914
- **VAN REENEN CA ; DICKS LMT.** Evaluation of Numerical Analysis of Random Amplified Polymorphic DNA (RAPD)-PCR as a Method to Differentiate Lactobacillus plantarum and Lactobacillus pentosus. *Cur. Microbiol.,* 1996, vol. 32, 183-187
- **VINDEROLA CG ; REINHEIMER JA.** Lactic acid starter and probiotic bacteria: a comparative ''in vitro'' study of probiotic characteristics and biological barrier resistance. *Food Res. Int.,* 2003, vol. 36, 895-904

SEQUENCE LISTING

<110> SELUR VK HOLDING EOOD [BG]

<120> New strains of lactic acid bacteria and their combinations producing probiotic preparations

<160> 5

<170> BiSSAP 1.2

<210> 1
<211> 1143
<212> DNA
<213> Lactobacillus gasseri

<220>
<221> source
<222> 1..1143
<223> /organism="Lactobacillus gasseri "
/mol_type="unassigned DNA"

<300>
<301> Metoda Zoriè Peternel, Andreja Ėanžek Majheniè, Helge Holo, Ingolf F. Nes, Zhian Salehian, Aleš Berlec, Irena Rogelj
<302> Wide-Inhibitory Spectra Bacteriocins Produced by Lactobacillus gasseri K7
<303> Probiotics and antimicrobial proteins
<304> Vol. 2
<305> Iss.4
<306> 233-340
<308> GenBank, EF392861
<309> 2010-02-23

<400> 1
aaatcttaga attagataat taaaaaaggt cgaaacttca tcttagtacg aagtttcgac 60

cctttttagt tatattttaa gctaaattag tcacttttcc tcttaagatt tattttttta 120

gtaaactata aaaaaatata aagaaaagga tataaacaaa catgaaagta ttaaacgagt 180

gtcaactaca aaccgtcgtt ggtggaaaaa attggtcagt tgcaaagtgc ggaggaacaa 240

ttggtactaa tatagctatt ggtgcatgga gaggtgcacg ggctggatcc ttttttggtc 300

agccagtttc tgtaggaaca ggtgcactaa tcggtgcaag tgctggtgca attggcggat 360

cagtacaatg tgtgggctgg ttagctggag gtggaagata atgatcgaaa aagtttctaa 420

aaatgaacta agccggatat atggtggaaa caacgtaaat tggggtagtg ttgcaggatc 480

atgtggtaaa ggtgcagtaa tggaaatata tttcgggaat cccatattag ggtgcgctaa 540

cggagctgca acatcattgg ttctacaaac tgctagtgga atatataaaa attatcaaaa 600

aaagagatag tatatgacgg gaatatggat agtaattatt agtattattg tgctttcaat 660

aattatcacc aatatagtag ctctaataca gacactatta cataagaaaa atgaaaaata 720

ttattttgat aaatcttttg gagcctatgc aggtaaaaat aatccaaaat atctgtttaa 780

taatgttgaa catcatgatt ttttccatgt attttatcag tattttattt gctcatatat 840

tccatttata gatgtcattt tttggttctt aggttcaatt tggtaataat caaaaaaggt 900

cgaaacttca tcttagtacg aagtttcgac cttttttat ttcaaattat ttttattgct 960

tccacgctgc atcaaatttt gctttacctg ctttaatttg attatcaaca ttttgaccat 1020

tcttagctgc atacaaaatc ttagccataa ttggatctaa ttgactgtag gcagcgtttg 1080

agttcttagc tactggaata ctgtacaagt gtttcatagc cccttctaac ttagctggaa 1140

gct 1143

<210> 2
<211> 3276
<212> DNA
<213> Lactobacillus gasseri

<220>
<221> source
<222> 1..3276
<223> /organism="Lactobacillus gasseri "
/mol_type="unassigned DNA"

<300>
<301> Metoda Zoriè Peternel, Andreja Èanžek Majhenič, Helge Holo, Ingolf F. Nes, Zhian Salehian, Aleš Berlec, Irena Rogelj
<302> Wide-Inhibitory Spectra Bacteriocins Produced by Lactobacillus gasseri K7
<303> Probiotics and antimicrobial proteins
<304> Vol. 2
<305> Iss.4
<306> 233-340
<308> GenBank, AY307382
<309> 2010-02-23

<400> 2
cccattttag gtcaaagatt aagtattgat ctaaatctgc gatacatcag gcatattttt 60

gaattaccaa tggaattctt tgtaacaaga agaacgggtg aaattacttc gcgtttttct 120

gatgcaagta gaattattga cgctttggca agtacagtta tttcgctctt cttagacctc 180

tcaattgtga ttgtgatggg attagtctta gcagcacaaa atatgacatt atttgggatt 240

acactgttag ctttgcctat ttatgcagtt gtaattctag gttttactaa aaagtttgaa 300

aaactaaata acgaacaaat ggaaagtaat gctgttttga gttcttcagt tattgaagat 360

attcaaggaa ttgaaactat taaagcttta aatagtgaga atacaagata tagaagaatc 420

gacagtcagt ttgtagatta cttaaagaag tcatttaaat atagtaagac tgagagttta 480

cagacagcac tcaagacatt tattcaatta tctcttaatg taattgttct ttgggttggt 540

gcaaagattg taatgcaagg acaattgagc attggtcagt taatgacgtt caatgcattg 600

ctggcatatt tcattgatcc cttgcaaagt attattaatt tacaaccgcg acttcaatca 660

gctagtgttg ctcaaaatcg attaaatgag gtctatcaag taaaaagtga attcaatcag 720

aaggctacta ttgaagatcg taagctccta gaaggaaata ttgaatataa gaatgtggat 780

tatagctatg gttatggcac agatgtctta aaagatatta atctcaaaat tagccagggc 840

gaaaaattaa cgattgttgg aatgagtggt tctggaaaat ctacgatggt taagttgtta 900

gtagatttct tttcgccaag taaaggtcag gttacattaa atggacatgc aacaagtgag 960

attgataagc atacgttgcg gtcatatgta aattatgtac ctcaaactcc atatatcttt 1020

tctggaacag ttaaagagaa tttgttgtta ggttgtaggc cagatatcac tgaagaagat 1080

gtaataaagg cttgtcaaat tgctgagatt gaccaagaaa tagctaatct acctttgcaa 1140
tttgaaacaa aattagatga aaatgccaaa attttatctg gtggacaaaa acaaaggtta 1200
actattgccc gggcattact atctcctgca aaggtattta tttttgatga agtaacaagt 1260
ggcttagata caattactga gaagaaagtg attgataatt tgatgaagct aaaggataag 1320
acaattatct ttattgcgca tcgactagca attgcagaac gggcagacaa ggtagttgta 1380
attgatcatg gtcaaatagt tgaagaaggc agccacagcg aattgatgag taagcatggt 1440
ttttactatg acttagtgaa gggataggtg agtacagatg gatgcaaaag agtatgaaag 1500
tacagaattc tattcctata agttcaagaa tttctcgaca atgattatta ttccagccgc 1560
tatttttgtt ctacttttat ttattggatc ttttttgct gttagacaga gtacagtctc 1620
ttcggttggc gttgttgaac caacggttgt aattaatcag aaaaatgtta gctatgatga 1680
gggacaagtc gttacaaaac atggtcaaaa atgggtagct catgttgatc aagatagtgg 1740
gattagttta atgcctgtta tgaaagctaa aggaaaagtg aggattgtta cctatgtgcc 1800
cactaataaa atctcgtcta ttaagaaggg gcagaagtta aacttttctg tacctacagt 1860
agacggcttg aatagtagat taactggtaa ggtaaaggag attggtgttt atccaataaa 1920
tgtgaacaag caaagtatgt atgaagttat ttcgattgcc aaagttagtg atataaatgt 1980
taagtacgga atgcaaggta atgccacaat agtgacaggt cgtagcacat attttaacta 2040
ttttttagat aaggtattga ataaaagata aatttgatag aataaaaaat taaaatttta 2100
ttaatctaaa gataaaaata taagtgaata aaaaagagat gttagataag aatattgatt 2160
tgcaaagagc aatatttcat ataaagcaag atattaattt gtattctgta gtgtatggat 2220
ttaaattgcc tgaaacttaa ttttgggaac gaatttggat aaaatatact gttttggtta 2280
gtaagtaagc aatattaatc catataaaaa ataaattgtt ttttatagta ggctaaatat 2340
ataaagatta ttaattatag gaggtcttta ttatgaaaaa ttttaataca ttatcatttg 2400
aaacattggc taacatagtt ggtgggagaa ataattgggc tgctaatata ggtggagtag 2460
gtggagcgac agtcgctgga tgggctcttg gaaatgcagt ttgcggtcct gcttgtggct 2520
ttgttggagc acactatgtt ccaatagcat gggctggcgt aacggcagct actggtggat 2580
tcggaaagat aagaaagtag ggaattaata tggctttaaa aacattagaa aaacatgaat 2640
taagaaatgt aatgggtgga aacaagtggg ggaatgctgt aataggagct gctacgggag 2700
ctactcgcgg agtaagttgg tgcagaggat tcggaccatg gggaatgact gcctgtgcgt 2760
taggaggtgc tgcaattgga ggatatctgg gatataagag taattaattt atatagagat 2820
ttatgttatt ctttgtaact attattaatg taatactttt tttaggtaac ttgtatcaaa 2880
cgggctttaa ttattttaac tttaatcaat tgcaagcact tttactaact atttttctta 2940
ttgttactgt aatagttatc gctattaata aaaaattaga aagaggttat gatctttctt 3000
tttttgcact ttatttgtat tggccaattt atgtttttga agttaaatta tataattcaa 3060
caaattataa agatggaata gaattttggg tttatttcgt actaattttg gccatatcgt 3120

ttgtaatagg aaaaatatta aaaagaatca taagaaagta ataataaaac tgaaagtact 3180

aaattagttt ttgaatgact gatttagtgc tttttctact ttctccttta tttaagtaaa 3240

tatgaaaatc taatagattt acttaatgtt aagctt 3276

<210> 3
<211> 619
<212> DNA
<213> Lactobacillus delbrueckii subsp. bulgaricus

<220>
<221> source
<222> 1..619
<223> /organism="Lactobacillus delbrueckii subsp. bulgaricus "
/mol_type="unassigned DNA"

<400> 3
gatttgttgg acgctagcgg cggatgggtg agtaacacgt gggcaatctg ccctaaagac 60

tgggatacca cttggaaaca ggtgctaata ccggataaca acatgaatcg catgattcaa 120

gtttgaaagg cggcgyaagc tgtcacttta ggatgagccc gcggcgcatt agctagttgg 180

tggggtaaag gcctaccaag gcaatgatgc gtagccgagt tgagagactg atcggccaca 240

ttgggactga gacacggccc aaactcctac gggaggcagc agtagggaat cttccacaat 300

ggacgcaagt ctgatggagc aacgccgcgt gagtgaagaa ggttttcgga tcgtaaagct 360

ctgttgttgg tgaagaagga tagaggcagt aactggtctt tatttgacgg taatcaacca 420

gaaagtcacg gctaactacg tgccagcagc cgcggtaata cgtaggtggc aagcgttgtc 480

cggatttatt gggcgtaaag cgagcgcagg cggaatgata agtctgatgt gaaagcccac 540

ggctcaaccg tggaactgca tcggaaactg tcattcttga gtgcagaaga ggagagtgga 600

attccatgtg tagcggtga 619

<210> 4
<211> 609
<212> DNA
<213> Streptococcus thermophilus

<220>
<221> source
<222> 1..609
<223> /organism="Streptococcus thermophilus"
/mol_type="unassigned DNA"

<400> 4
gagttgcgaa cgggtgagta acgcgtaggt aacctgcctt gtagcggggg ataactattg 60

gaaacgatag ctaataccgc ataacaatga atgactcatg tcatttattt gaaaggggca 120

attgctccac tacaagatgg acctgcgttg tattagctag taggtgaggt aacggctcac 180

ctaggcgacg atacatagcc gacctgagag ggtgatcggc cacactggga ctgagacacg 240

gcccagactc ctacgggagg cagcagtagg gaatcttcgg caatgggggc aaccctgacc 300

gagcaacgcc gcgtgagtga agaaggtttt cggatcgtaa agctctgttg taagtcaaga 360

acgagtgtga gagtggaaag ttcacactgt gacggtagct taccagaaag ggacggctaa 420

```
ctacgtgcca gcagccgcgg taatacgtag gtcccgagcg ttgtccggat ttattgggcg     480
taaagcgagc gcaggcggtt tgataagtct gaagttaaag gctgtggctc aaccatagtt     540
cgctttggaa actgtcaaac ttgagtgcag aaggggagag tggaattcca tgtgtagcgg     600
tgaaatgcg                                                             609
```

```
<210> 5
<211> 645
<212> DNA
<213> Lactobacillus gasseri

<220>
<221> source
<222> 1..645
<223> /organism="Lactobacillus gasseri"
      /mol_type="unassigned DNA"

<400> 5
aatttggtgc ttgcaccaaa tgaaactaga tacaagcgag cggcggacgg gtgagtaaca      60
cgtgggtaac ctgcccaaga gactgggata acacctggaa acagatgcta ataccggata     120
acaacactag acgcatgtct agagtttaaa agatggttct gctatcactc ttggatggac     180
ctgcggtgca ttagctagtt ggtaaggtaa cggcttacca aggcaatgat gcatagccga     240
gttgagagac tgatcggcca cattgggact gagacacggc ccaaactcct acgggaggca     300
gcagtaggga atcttccaca atggacgcaa gtctgatgga gcaacgccgc gtgagtgaag     360
aagggtttcg gctcgtaaag ctctgttggt agtgaagaaa gatagaggta gtaactggcc     420
tttatttgac ggtaattact tagaaagtca cggctaacta cgtgccagca gccgcggtaa     480
tacgtaggtg gcaagcgttg tccggattta ttgggcgtaa agcgagtgca ggcggttcaa     540
taagtctgat gtgaaagcct tcggctcaac cggagaattg catcagaaac tgttgaactt     600
gagtgcagaa gaggagagtg gaactccatg tgtagcggtg aaatg                     645
```